# EUROPEAN PATENT SPECIFICATION

(11) **EP 4 554 464 B1**
(45) Date of publication and mention of the grant of the patent: **14.10.2026**
(21) Application number: 23754011.7
(22) Date of filing: 13.07.2023
(51) Int. Cl.: A61B 5/0215, A61B 5/00

(54) **SYSTEMS AND METHODS OF FOR COMPUTER-ASSISTED MEASUREMENT OF PULMONARY CAPILLARY WEDGE PRESSURE**
SYSTEME UND VERFAHREN ZUR MASCHINENLERNEN-GESTÜTZTEN MESSUNG DES PULMONALEN KAPILLAREN WEDGE-DRUCKS
SYSTEMES ET METHODES POUR LA MESURE DE LA PRESSION CAPILLAIRE PULMONAIRE A L'AIDE DE L'APPRENTISSAGE AUTOMATIQUE

(30) Priority: 14.07.2022 US 202263389331 P; 24.02.2023 US 202363486914 P
(43) Date of publication of application: 21.05.2025
(73) Proprietor: Becton, Dickinson and Company, Franklin Lakes, NJ 07417 (US)
(72) Inventor: ANGEL, Nathan, Alexander, Irvine, CA 92614 (US); AL HATIB, Feras, Irvine, CA 92614 (US); KONNO, Mark, A., Irvine, CA 92614 (US); JIAN, Zhongping, Irvine, CA 92614 (US)
(74) Representative: Venner, Julia Ann
(86) International application number: PCT/US2023/027678
(87) International publication number: WO 2024/015533

(56) References cited:
- US-A1- 2020 205 739
- BOOTSMA I T ET AL: "The contemporary pulmonary artery catheter. Part 1: placement and waveform analysis", JOURNAL OF CLINICAL MONITORING AND COMPUTING, SPRINGER NETHERLANDS, DORDRECHT, vol. 36, no. 1, 10 February 2021 (2021-02-10), pages 5 - 15, XP037708047, ISSN: 1387-1307, [retrieved on 20210210], DOI: 10.1007/S10877-021-00662-8
- FETANAT MASOUD ET AL: "Fully Elman Neural Network: A Novel Deep Recurrent Neural Network Optimized by an Improved Harris Hawks Algorithm for Classification of Pulmonary Arterial Wedge Pressure", IEEE TRANSACTIONS ON BIOMEDICAL ENGINEERING, vol. 69, no. 5, 23 November 2021 (2021-11-23), USA, pages 1733 - 1744, XP093085515, ISSN: 0018-9294, Retrieved from the Internet <URL:https://ieeexplore.ieee.org/stampPDF/getPDF.jsp?tp=&arnumber=9625749&ref=aHR0cHM6Ly9pZWVleHBsb3JlLmllZWUub3JnL2Fic3RyYWN0L2RvY3VtZW50Lzk2MjU3NDk=> DOI: 10.1109/TBME.2021.3129459

## Description

### CROSS-REFERENCE TO RELATED APPLICATIONS

The current application claims priority to U.S. Provisional Patent Application No. 63/389,331, filed July 14, 2022, entitled "Systems and Methods for Machine Learning Enabled Measurement of Pulmonary Capillary Wedge Pressure" to Al Hatib, et al. and U.S. Provisional Patent Application No. 63/486,914, filed February 24, 2023, entitled "Systems and Methods for Computer Enabled Measurement of Pulmonary Capillary Wedge Pressure" to Angel, et al.

### TECHNOLOGICAL FIELD

The present invention generally relates to systems for computer-assissted acquisition and analysis of pulmonary capillary wedge pressure (PCWP).

### BACKGROUND

Left atrial pressure is an important measuement in individuals possessing dysfunction in the left ventricle and/or posssessing a valvular disesase. While direct methods of determining left atrial pressure are possible, such methods (e.g., a transseptal approach) possess inherent risks. As such, indirect methods are typically used, including determining pulmonary capillary wedge pressure (PCWP), also referred to as pulmonary artery occlusion pressure (PAOP) using a Pulmonary Artery Cather (PA catheter), also referred to as Swan-Ganz catheter.

Although PCWP measurements are deemed a low-risk procedure, accurate interpretation of pulmonary artery pressure (PAP) and PCWP waveforms often require specialists. Thus, there is a need for methods and systems that can assist in ensuring accurate acquisition and analysis of PAP and PCWP measurements.

Bootsma, I.T, et al: "The contemporary pulmonary artery catheter. Part 1: placement and waveform analysis", JOURNAL OF CLINICAL MONITORING AND COMPUTING, SPRINGER NETHERLANDS, DORDRECHT, vol. 36, no.1, 10 February 2021, pages 5-15 introduces the difference between intermittent pulmonary artery thermodilution using cold bolus injections, and the contemporary PAC enabling continuous measurements by using a thermal filament which at random heats up the blood. The insertion techniques, interpretation of waveforms of the PAC, the interaction of waveforms with the respiratory cycle and airway pressure as well as pitfalls in waveform analysis are discussed. Also discussed are the measurements of the contemporary PAC including measurement of continuous cardiac output, RV ejection fraction, end-diastolic volume index, and mixed venous oxygen saturation. Limitations of all of these measurements are highlighted. It is concluded that thorough understanding of measurements obtained from the PAC are the first step in successful application of the PAC in daily clinical practice.

Masoud, Fetanat et al: "Fully Elman Neural Network: A Novel Deep Recurrent Neural Network Optimized by an Improved Harris Hawks Algorithm for classification of Pulmonary Arterial Wedge Pressure", IEEE Transactions on Biomedical Engineering, vol.69, no.5, 23 November 2021, pages 1733-1744, describes, firstly, an improved Harris Hawks optimizer algorithm called HHO+ which is presented and tested on 24 unimodal and multimodal benchmark functions. Secondly, a novel fully Elman neural network (FENN) is proposed to improve the classification performance. Finally, four novel 18-layer deep learning methods of convolutional neural networks (CNNs) with multi-layer perceptron (CNN-MLP), CNN with Elman neural networks (CNN-ENN), CNN with fully Elman neural networks (CNN-FENN), and CNN with fully Elman neural networks optimized by HHO+ algorithm (CNN-FENN-HHO+) for classification of abnormal and normal PAWP using estimated HVAD pump flow were developed and compared. The estimated pump flow was derived by a non-invasive method embedded into the commercial HVAD controller. The proposed methods are evaluated on an imbalanced clinical dataset using 5-fold cross-validation. It is disclosed that the proposed CNN-FENN-HHO+ method outperforms the proposed CNN-MLP, CNN-ENN and CNN-FENN methods and improved the classification performance metrics across 5-fold cross-validation with an average sensitivity of 79%, accuracy of 78% and specificity of 76%. It is discussed that the proposed methods can reduce the likelihood of hazardous events like pulmonary congestion and ventricular suction for HF patients and notify identified abnormal cases to the hospital, clinician and cardiologist for emergency action, which can diminish the mortality rate of patients with HF.

US 2020/205739 describes systems and methods for the quantification of the quality of an acquired signal for assessment and for gating the acquired signal for subsequent analysis. A signal is acquired, and a determination is made in real-time if there is a problem with the acquisition (e.g., if the acquired signal is acceptable or unacceptable; is of sufficient quality for subsequent assessment). If there is a problem, output is provided via the systems and methods described herein to indicate that signal acquisition needs to be performed again (e.g., if the acquired signal is unacceptable, reject the acquired signal and acquire a new signal).

### SUMMARY OF THE INVENTION

The invention is defined in the appended set of claims.

### SUMMARY OF THE DISCLOSURE

In some implementations useful for understanding the presently claimed invention, a computational method (not part of the claimed invention) is for performing and assessing quality of a pulmonary capillary wedge pressure (PCWP) measurement of an individual. The method comprises acquiring a blood pressure waveform of an individual, wherein the waveform comprises a blood pressure measurement acquired from a pulmonary artery position and a blood pressure measurement acquired from a wedge position. The blood pressure waveform is acquired using a pulmonary artery catheter. The method comprises determining, using a computational processing system, a PCWP measurement from the blood pressure measurement from the wedge position. The method comprises determining, using the computational processing system, a quality assessment for the PCWP measurement using a machine-learning model configured to provide a quality assessment for the PCWP measurement based on the blood pressure measurement acquired from the pulmonary artery position and the blood pressure measurement acquired from the wedge position.

In the presently claimed invention, a hemodynamic monitoring system is for performing and assessing quality of a pulmonary capillary wedge pressure (PCWP) measurement. The system comprises a pulmonary artery catheter configured to acquire blood pressure measurement acquired from a pulmonary artery position and a blood pressure measurement acquired from a wedge position. The pulmonary artery catheter comprises a balloon that is inflatable. The system comprises a computational processing system in connection with the pulmonary artery catheter. The computational system comprises a processor system, a display screen in digital connection with the processor system, and a memory that comprises one or more applications. The one or more applications can direct the processor to acquire a blood pressure measurement in a pulmonary artery position. The one or more applications can direct the processor to inflate the balloon. Inflating the balloon allows the catheter to migrate to a wedge position. The one or more applications can direct the processor to acquire a blood pressure measurement in the wedge position. The one or more applications can direct the processor to generate a blood pressure waveform from blood pressure measurement acquired from a pulmonary artery position and a blood pressure measurement acquired from a wedge position. The one or more applications can direct the processor to determine a PCWP measurement from the blood pressure measurement from the wedge position. The one or more applications can direct the processor to determine a quality assessment for the PCWP measurement using a machine-learning model configured to provide a quality assesment for the PCWP measurement based on the blood pressure measurement acquired from the pulmonary artery position and the blood pressure measurement acquired from the wedge position. The one or more applications can direct the processor to display one or more of: the blood pressure waveform, the PCWP measurement, or the quality assessment for the PCWP measurement on the display screen.

In some implementations, the blood pressure waveform is generated and displayed on the display screen in real time.

In some implementations, the PCWP measurement is determined and displayed on the display screen in real time.

In some implementations, the quality assessment for the PCWP measurement is determined and displayed on the display screen in real time.

In some implementations useful for understanding the presently claimed invention, a computational method (not part of the claimed invention) for performing a pulmonary capillary wedge pressure (PCWP) measurement of an individual. The method comprises acquiring, using a the pulmonary artery catheter, a blood pressure waveform of an individual. The waveform comprises a blood pressure measurement acquired from a pulmonary artery position. The pulmonary artery catheter is in connection with a hemodynamic monitoring system. The method comprises assessing, using the hemodynamic monitoring system, the blood pressure measurement acquired from a pulmonary artery position for artifacts while blood pressure measurement is being acquired from the pulmonary artery position. The method comprises upon a determination that the blood pressure measurement acquired from a pulmonary artery position is devoid of artifacts, inflating, using the hemodynamic monitoring system, a balloon at or near the distal end of the pulmonary artery catheter to allow the pulmonary artery catheter to migrate to the wedge position. The method comprises further acquiring, using the pulmonary artery catheter, a blood pressure waveform of an individual, wherein the waveform comprises a blood pressure measurement acquired from a the wedge position. The method comprises determining, using the hemodynamic monitoring system, a PCWP measurement from the blood pressure measurement from the wedge position.

In some implementations useful for understanding the presently claimed invention, a hemodynamic monitoring system is for performing a pulmonary capillary wedge pressure (PCWP) measurement. The system comprises a pulmonary artery catheter configured to acquire blood pressure measurement acquired from a pulmonary artery position and a blood pressure measurement acquired from a wedge position. The pulmonary artery catheter comprises a balloon that is inflatable. The system comprises a computational processing system in connection with the pulmonary artery catheter. The computational system comprises a processor system, a display screen in digital connection with the processor system, and a memory system comprising one or more applications. The one or more applications that can direct the processor system to acquire a blood pressure measurement in a pulmonary artery position. The one or more applications that can direct the processor system to assess the blood pressure measurement acquired from a pulmonary artery position for artifacts. The one or more applications that can direct the processor system to upon a determination that the blood pressure measurement acquired from a pulmonary artery position is devoid of artifacts, inflate the balloon, wherein inflating the balloon allows the catheter to migrate to a wedge position. The one or more applications that can direct the processor system to acquire a blood pressure measurement in the wedge position. The one or more applications that can direct the processor system to generate a blood pressure waveform from blood pressure measurement acquired from a pulmonary artery position and a blood pressure measurement acquired from a wedge position. The one or more applications that can direct the processor system to determine a PCWP measurement from the blood pressure measurement from the wedge position. The one or more applications that can direct the processor system to display the PCWP measurement on the display screen.

In some implementations useful for understanding the presently claimed invention, a computational method for detecting a transition between a pulmonary artery position and a wedge position. The method comprises acquiring, using a the pulmonary artery catheter, a blood pressure waveform of an individual. The waveform comprises a blood pressure measurement acquired from a pulmonary artery position. The pulmonary artery catheter is in connection with a hemodynamic monitoring system. The method comprises inflating, using the hemodynamic monitoring system, a balloon at or near the distal end of the pulmonary artery catheter to allow the pulmonary artery catheter to migrate to the wedge position. The method comprises detecting, using the hemodynamic monitoring system, a transition from a pulmonary artery position to a wedge position. The method comprises further acquiring, using the pulmonary artery catheter, a blood pressure waveform of an individual. The waveform comprises a blood pressure measurement acquired from a the wedge position. The method comprises deflating, using the hemodynamic monitoring system, the balloon at or near the distal end of the pulmonary artery catheter to allow the pulmonary artery catheter to migrate back to the pulmonary artery position. The method comprises detecting, using the hemodynamic monitoring system, a transition from a wedge position to a pulmonary artery position.

In some implementations useful for understanding the presently claimed invention, a hemodynamic monitoring system is for detecting a transition between a pulmonary artery position and a wedge position. The system comprises a pulmonary artery catheter configured to acquire blood pressure measurement acquired from a pulmonary artery position and a blood pressure measurement acquired from a wedge position. The pulmonary artery catheter comprises a balloon that is inflatable. The system comprises a computational processing system in connection with the pulmonary artery catheter. The computational processing system comprises a processor system, a display screen in digital connection with the processor system, and a memory system comprising one or more applications. The one or more applications can direct the processor system to acquire a blood pressure measurement in a pulmonary artery position. The one or more applications can direct the processor system to inflate the balloon. Inflating the balloon allows the catheter to migrate to a wedge position. The one or more applications can direct the processor system to detect a transition from a pulmonary artery position to a wedge position. The one or more applications can direct the processor system to acquire a blood pressure measurement in the wedge position. The one or more applications can direct the processor system to deflate the balloon at or near the distal end of the pulmonary artery catheter to allow the pulmonary artery catheter to migrate back to the pulmonary artery position. The one or more applications can direct the processor system to detect a transition from a wedge position to a pulmonary artery position.

In some implementations, the PCWP measurement and the quality assessment are each determined in real time.

In some implementations, the computational processing system and the pulmonary artery catheter are part of a hemodynamic monitoring system.

In some implementations, acquiring the blood pressure waveform comprises inserting the pulmonary artery catheter into a central vein of the individual. Acquiring the blood pressure waveform comprises guiding the pulmonary artery catheter to the pulmonary artery. Acquiring the blood pressure waveform comprises inflating a balloon at or near the distal end of the pulmonary artery catheter to allow the pulmonary artery catheter to migrate to the wedge position.

In some implementations, the method comprises prior to inflating a balloon, assessing in real time, using the computational processing system, the blood pressure measurement acquired from a pulmonary artery position for artifacts.

In some implementations, artifacts comprise one or more of: measurements obtained during patient movement, measurements obtained during catheter flushing, and measurements indicating a flat line, measurements indicating underdamped, and measurements indicating overdamped.

In some implementations, detection of an artifact based on flushing, improper zero point, or patient movement is detected if the PAP maximum (*PAPₘₐₓ*) is greater than a threshold, if the PAP minimum (*PAPₘᵢₙ*) is less than threshold, and/or if the PAP minimum subtracted from the PAP maximum (*PAPₘₐₓ - PAPₘᵢₙ*) is greater than threshold; wherein PAP is the blood pressure measurement acquired from a pulmonary artery position.

In some implementations, detection of an artifact based on a flat line or overdamping is detected if the PAP minimum subtracted from the PAP maximum (*PAPₘₐₓ - PAPₘᵢₙ*) is less than threshold; wherein PAP is the blood pressure measurement acquired from a pulmonary artery position.

In some implementations, the pulmonary artery catheter comprises a lumen configured to measure the blood pressure.

In some implementations, the pulmonary artery catheter is a Swan-Ganz catheter.

In some implementations, the machine-learning model is based on at least one feature selected from the group consisting of: a waveform phase feature, a determinable feature, and a morphological feature. The waveform phase feature is selected from: contractility, pulmonary artery compliance, stroke volume, vascular tone, afterload, and full cardiac cycle. The determinable feature is determined based on a waveform phase feature and is selected from: mean of the phase, a maximum, a minimum, a duration, an area, a standard deviation, a slope, a derivative, a trend, a deviation from a trend, a variation, and a variance. The morphological feature is based on a waveform feature and is selected from: a frequency component, skewness, and kurtosis.

In some implementations, the machine-learning model is based on one or more of: approximate entropy, sample entropy, baroreflex sensitivity, variability, and changes.

In some implementations, acquiring the PCWP measurement comprises determining respiratory cycles of the individual. The determining the PCWP measurement is based on the respiratory cycles of the individual.

In some implementations, the determining of the PCWP comprises determining a blood pressure measurement from the wedge position at the end of expiration of a respiratory cycle.

In some implementations, the determining of the PCWP comprises averaging blood pressure measurement from the wedge position across one or more respiratory cycles.

In some implementations, the method comprises detecting in real time, using the computational processing system, a transition from a pulmonary artery position to a wedge position or from a wedge position to a pulmonary artery position by: extracting one or more hemodynamic features from the blood pressure measurement in the pulmonary position and the blood pressure measurement in the wedge position and determining, using a fuzzy logic membership function, a fuzzy logic value based on a change in value between the blood pressure measurement in the pulmonary position and the blood pressure measurement in the wedge position for each hemodynamic feature of the one or more hemodynamic features.

In some implementations, the one or more features comprise at least one of: a statistical moment, a percentile value of data, histogram of data, diastolic pressure, median pressure, mean pressure, systolic pressure, pulse pressure, Shannon Entropy, Number of peaks above a percentile, number of valleys below a percentile, number of mean crossings, area under curve, singular vector coefficient after principle components analysis, a subsampled signal, a determinable feature, a waveform phase feature, and a morphological feature.

In some implementations, the morphological feature is selected from: a frequency component, skewness, and kurtosis; wherein the waveform phase feature is selected from: contractility, pulmonary artery compliance, stroke volume, vascular tone, afterload, and full cardiac cycle; and wherein the determinable feature is selected from: a mean of the phase, a maximum, a minimum, a duration, an area, a standard deviation, a slope, a derivative, a trend, a deviation from a trend, a variation, and a variance.

In some implementations, the one or more features comprise mean pressure and pulse pressure.

In some implementations, the method further comprises segmenting the blood pressure measurement acquired from the pulmonary artery position and the blood pressure measurement acquired from the wedge position into temporal windows. The method further comprises extracting features from the temporal windows of the blood pressure measurement acquired from the pulmonary artery position and the blood pressure measurement acquired from the wedge position. The machine-learning model is trained to detect whether an extracted feature of a temporal window is derived the blood pressure measurement acquired from the pulmonary artery position or blood pressure measurement acquired from the wedge position. Determining, using the computational processing system, a quality assessment for the PCWP measurement using a machine-learning model comprises entering the extracted features from the temporal windows into the machine-learning model to yield a quality assessment for each temporal window. The quality assessment is based on whether the extracted features for each temporal window can be differentiated as derived from the pulmonary artery position or from the wedge position.

In some implementations, the quality assessment is categorical.

In some implementations, the categories are a qualitative ranking.

In some implementations, determining, using the computational processing system, a quality assessment for the PCWP measurement using a machine-learning model comprises determining whether one or more extracted features of a temporal window are above or below a threshold.

In some implementations, wherein the one or more extracted features comprise: *PCWP_{Mean}, PAP_{Diastolic}, and PCWP_{PulsePress},* wherein *PCWP_{Mean}* is a mean value of pressure measurements acquired in the wedge position; wherein *PAP_{Diastolic}* is diastolic pressure in the pulmonary artery position; and wherein *PCWP_{PulsePress}* is pulse pressure in the wedge position.

In some implementations, a high quality is indicated when: *PCWP_{Mean} < PAP_{Diastolic}* AND *a* × *PCWP_{PulsePress} + b* × *PCWP_{Mean}* ≤ *c*, wherein *a, b, and c are* determinant values.

In some implementations, a medium quality is indicated when: *PCWP_{Mean} < PAP_{Diastolic}* AND *a* × *PCWP_{PulsePress} + b* × *PCWP_{Mean}* > *c*, wherein *a, b, and c are* determinant values.

In some implementations, a low quality is indicated when: *PCWP_{Mean}* ≥ *PAP_{Diastolic}*.

In some implementations, the method further comprises assigning, using the computational processing system, each temporal window its quality assessment.

In some implementations, the method further comprises displaying the quality assessment of one or more temporal windows on a display screen in digital communication with the computational processing system.

In some implementations, determining, using a computational processing system, a PCWP measurement comprises averaging blood pressure measurement acquired from a wedge position of temporal windows that are determined to have a quality above a threshold.

In some implementations, determining, using a computational processing system, a PCWP measurement comprises excluding blood pressure measurement acquired from a wedge position of temporal windows that are determined to have a quality below a threshold and averaging non-excluded blood pressure measurement acquired from a wedge position of temporal windows.

In some implementations, the method further comprises displaying the quality assessment of one or more temporal windows on a display screen in digital communication with the computational processing system.

Additional implementations and features are set forth in part in the description that follows, and in part will become apparent to those skilled in the art upon examination of the specification or may be learned by the practice of the disclosure. A further understanding of the nature and advantages of the present disclosure may be realized by reference to the remaining portions of the specification and the drawings, which forms a part of this disclosure

### BRIEF DESCRIPTION OF THE DRAWINGS

The description will be more fully understood with reference to the following figures, which are presented as exemplary implementations of the invention and should not be construed as a complete recitation of the scope of the invention, wherein:
Figure 1 provides an example of a catheter located in a wedge position.
Figures 2A-2D provide an example of PA catheter positions within a heart and pressures obtainable by a PA catheter in such positions.
Figures 3A-3B provide examples of wedge positions with a proper (Figure 2A) wedge placement and a wedge position with an overinflated balloon (Figure 2B).
Figures 3C-3D provide examples of waveforms during PCWP measurement.
Figures 4A-4B provide an example of a method and computational processes acquiring and assessing quality of PWCP measurements.
Figure 5 provides an example of a computational method for detecting artifacts in a PAP waveform.
Figure 6 provides an example of a computational method for determining a transition point of balloon inflation or of balloon deflation.
Figure 7A-7E provides examples of utilizing a fuzzy logic membership function plots.
Figure 8 provides an example of a method to generate fuzzy logic membership functions.
Figure 9 provides an example of clinical data of a cohort of patients plotting pulse pressure against mean pressure.
Figure 10 provides an example of a computational method to perform PCWP measurement and quality assessment.
Figures 11A-11B provide examples of quality metrics.
Figure 12 provides an example of plotting quality assessment over wedge position waveforms.
Figures 13A-13B provide examples of pressure wave.
Figures 14A-14F provide example of various parameters derived from a pressure wave.
Figure 15 provides an example of computational processing system.
Figure 16 provides an example of a hemodynamic monitoring system.

### DETAILED DESCRIPTION

Turning now to the drawings, systems and methods for computer-assisted acquisition and analysis of pulmonary capillary wedge pressure (PCWP) are described. In many instances, a hemodynamic monitoring system comprising a pulmonary artery catheter system (e.g., Swan-Ganz catheter) is utilized to acquire a PCWP. Computational methods can be performed to assist and analyze the acquisition of the PCWP. In some instances, a computational method is utilized to detect when the pulmonary artery catheter is ready to acquire the PCWP (e.g., ready to inflate balloon on pulmonary catheter end). In some instances, a computational method is utilized to detect the balloon inflation point and/or to edtect the balloon deflation point. In some instances, a computaitonal method is utilized to detect respiratory signals and/or perform the PCWP measurement. In some instances, a trained computational model is utilized to assess the quality of the acquistion of the PCWP. In some instances, a computational system is configured to run one or more of the computational methods described. The computational system may be part of the hemodynamic monitoring system or utilized in conjunction with the hemodynamic monitoring system.

PCWP is frequently used to assess left ventricular filling, to represent left atrial pressure, and to assess mitral valve function. In many instances, the PCWP is also an estimate of left ventricular end-diastolic pressure (LVEDP). A physiological normal PCWP is between 4 to 12 mmHg, and elevated levels of PCWP might indicate severe left ventricular failure or severe mitral stenosis.

To measure PCWP, a balloon-tipped, multi-lumen catheter (e.g., a Swan-Ganz catheter) can be inserted through a central vein (such as femoral, subclavian, internal jugular, or any other suitable vein) and advanced through the superior or inferior vena cava to reach the right atrium. From the right atrium, the catheter can be advanced through the tricuspid valve into the right ventricle. Once in the right ventricle, the balloon at the tip of the catheter is inflated to advance the catheter to the right ventricular outflow tract, then to the pulmonary artery after crossing the pulmonic valve. Once the catheter is in the main pulmonary artery, the balloon is deflated. The tip of the catheter then lies in the main pulmonary artery, where the balloon can be reinflated as needed, which allows the catheter to migrate downstream (e.g., through the circulatory system in the direction of blood flow), where the balloon can occlude a branch of the pulmonary artery-i.e., a "wedge" position. The catheter can then provide a measurement of the PCWP, which should be equivalent to the pressure of the left atrium.

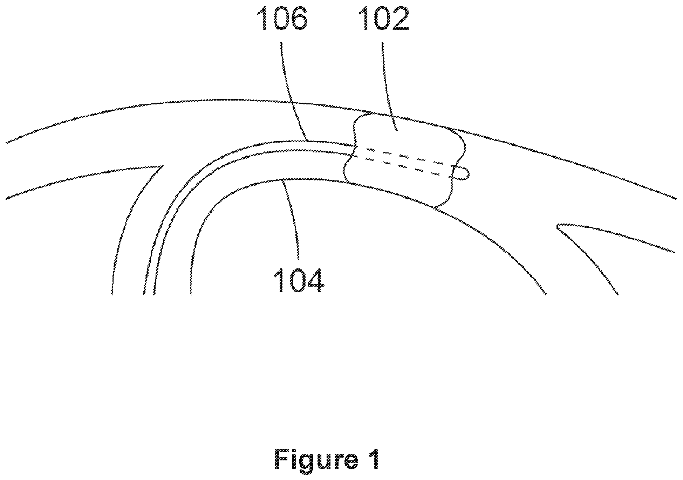

Figure 1 provides an exemplary illustration of a pulmonary artery catheter located in a wedge position. An inflated balloon 102 occludes a branch of pulmonary artery 104, to allow one or more ports located on a catheter 106 to obtain a pressure measurement. Pressure measurements can be a single measurement, continuous measurement, continual measurements, and combinations thereof.

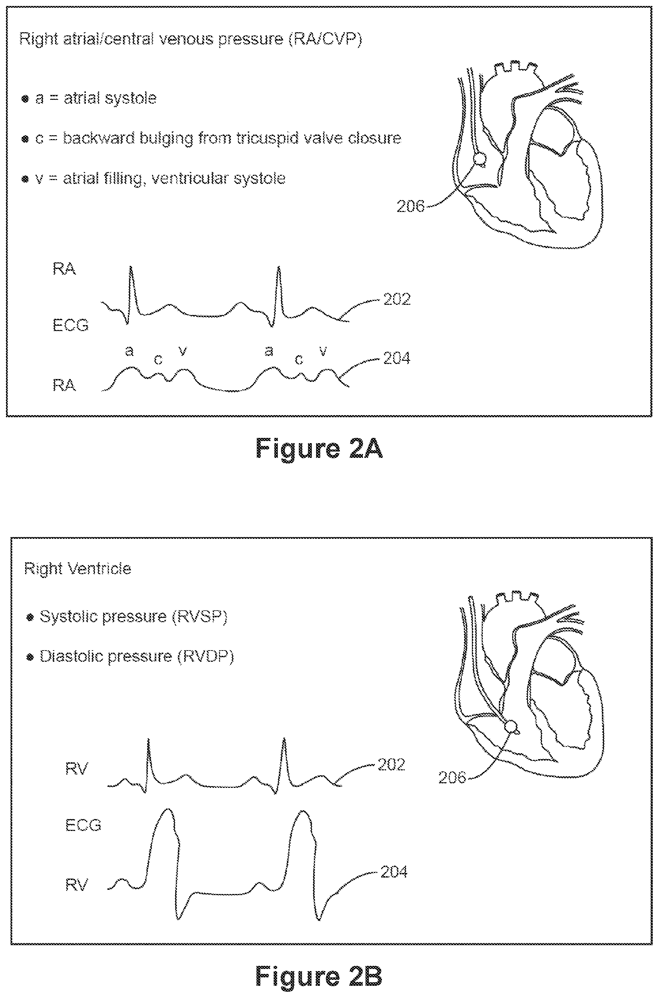

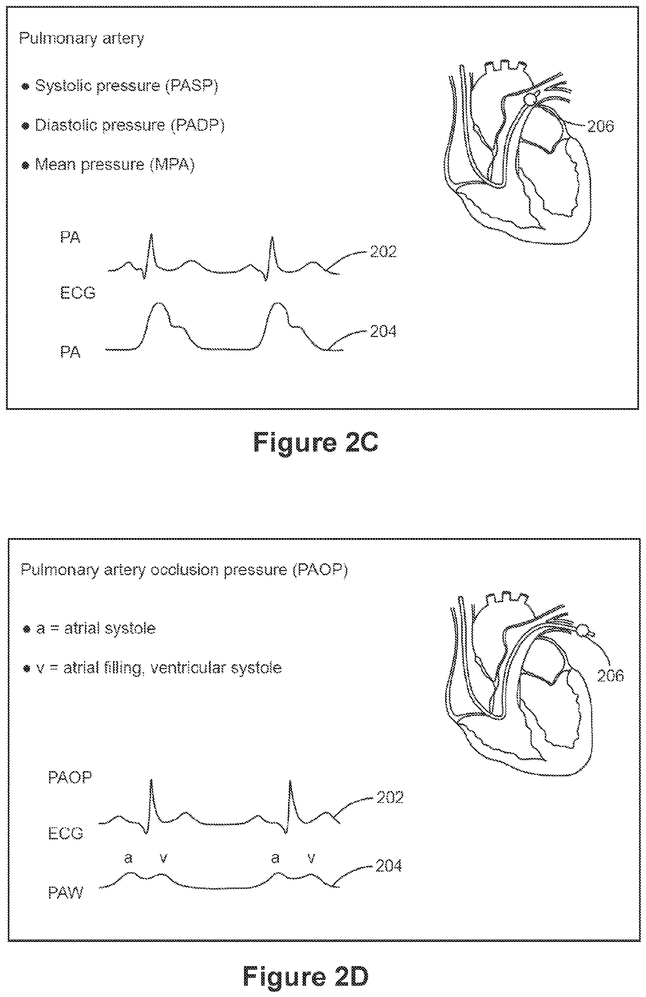

As a catheter is advanced through chambers of the heart and/or circulatory system, various blood pressure waveforms can be obtained and/or allow for specific measurements within that chamber, vessel, or other circulatory component. Figures 2A-2D provide examples of waveforms demonstrating progression of a catheter through the path from right atrium (Figure 2A) to a wedge position (Figure 2D). Figure 2A illustrates pressure obtained in the right atrium, in which a physiologically normal pressure is about 2-6 mmHg and a mean of 4 mmHg. Waveform 202 illustrates an exemplary ECG of the individual during a PCWP procedure, while waveform 204 provides an exemplary right atrial pressure waveform measured by the catheter 206 (e.g., a Swan-Ganz catheter). Peaks a, c, and v represent atrial systole, backward bulging from tricuspid valve closure, and atrial filling ventricular systole, respectively.

In Figure 2B, catheter 206 is advanced to the right ventricle, which causes a pattern change in pressure waveform 204. As catheter 206 is in the right ventricle, right ventricle systolic pressure (RVSP) and a right ventricle diastolic pressure (RVDP) can be measured, in which a physiologically normal RVSP is about 15-25 mmHg and a physiologically normal RVDP is about 0-8 mmHg.

Figure 2C illustrates an example of a pressure waveform 204 as catheter 206 is advanced into the pulmonary artery, where pulmonary artery systolic pressure (PASP), pulmonary artery diastolic pressure (PADP), and a mean pulmonary artery pressure (MPA) can be measured, in which a physiologically normal PASP is about 15-25 mmHg, physiologically normal PADP is about 8-15 mmHg, and physiologically normal MPA is about 10-20 mmHg.

Figure 2D illustrates an example of a pressure waveform 204 generated at the wedge position to yield a PCWP (also referred to as the pulmonary artery occlusion pressure (PAOP)). As noted previously, PCWP is tantamount to the left atrial pressure, in which a physiological normal PCWP is about 6-12 mmHg. Additionally, the pressure waveform 204 indicates peaks a and v, representing atrial systole and atrial filling ventricular systole, respectively.

Abnormal waveforms can arise for a variety of reasons, especially incorrect catheter placement. For example, placement too proximal to the right ventricle, placement too distal from the right ventricle, and/or failure of the catheter to migrate from the pulmonary artery into the wedge position. Additional abnormalities can occur from improper inflation of a balloon, including over- and under-inflation. Non-clinicians (e.g., nurses, doctors, physicians, surgeons, and/or other medical practitioners) may not recognize abnormal waveforms, which can lead to incorrect measurement interpretation or forgoing the procedure entirely.

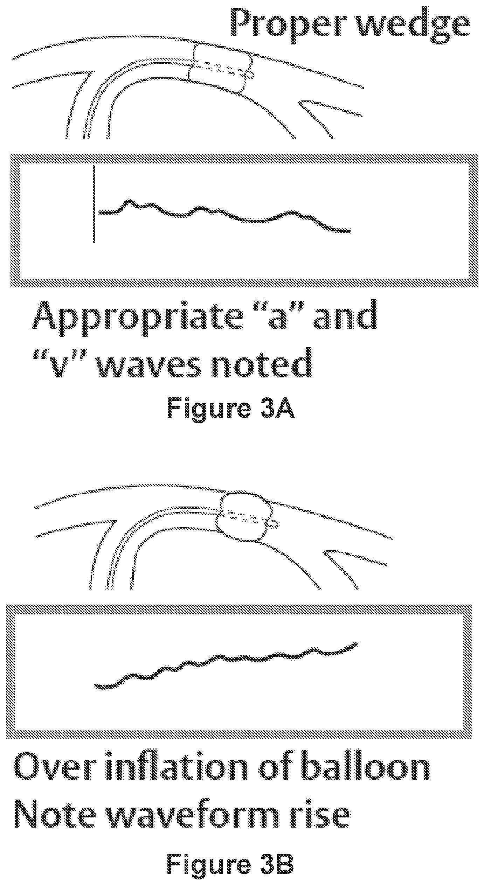

Figures 3A and 3B provide examples of pressure waveforms of correct and incorrect PCWP measurement. Specifically, Figure 3A illustrates a proper wedge, where the waveform indicates appropriate a and v waves, such as exemplified in Figure 2D. Figure 3B, however, depicts a waveform generated from a balloon that has been overinflated, resulting in a inaccurate PCWP waveform (indicated by the gradual rise the waveform) with less obvious a and v peaks.

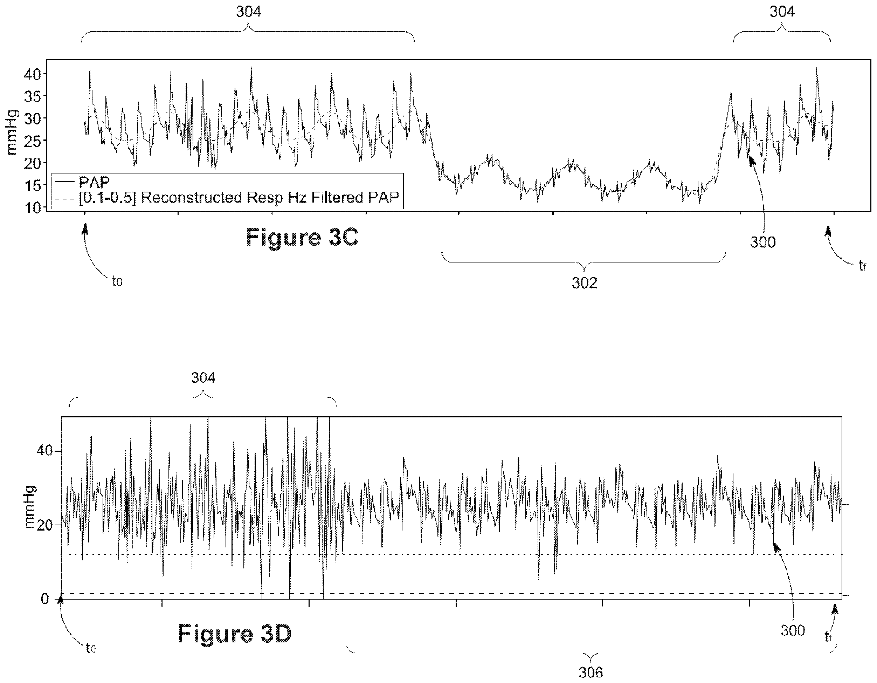

Figures 3C and 3D provide examples of pressure waveforms generated from a PCWP procedure. An example of an accurately acquired waveform is provided in Figure 3C, while an inaccurately acquired waveform is illustrated in Figure 3D.

In Figure 3C, waveform 300 illustrates a pressure measurement obtained by a medical provider beginning at time, t₀, and ending at time, t_{f}. Region 302 represents acquisition of pressure when the catheter is in the wedge position and the resultant PCWP waveform, while regions 304 represents acquisition of pressure when the catheter is in a pulmonary artery position (e.g., pre- and post-PCWP). In contrast, Figure 3D illustrates a situation in which waveform 300 possesses a dampened measurement in region 306. Such dampening can be caused by improper placement of a catheter or other issues during acquisition. To overcome these complications, hemodynamic monitoring systems as described herein can utilize one or more computational processes to ensure a proper wedge pressure reading. These systems and methods can be deployed in a medical facility to allow a clinician (e.g., doctor, physician, nurse, cardiologist, etc.) to acquire PCWP readings and assess their quality in real time. In some implementations, a hemodynamic monitoring system can indicate to a clinician the quality and/or whether the catheter needs to be repositioned and measurements reobtained.

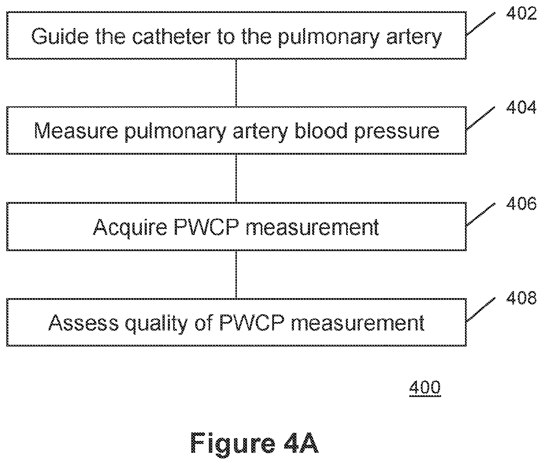

Provided in Fig. 4A is an example of a method 400 for performing a PCWP measurement. Generally, the method includes the use of a pulmonary artery catheter (e.g., Swan-Ganz catheter) that is delivered to the pulmonary artery to perform a PCWP measurement. The pulmonary artery catheter can comprise one or more sensors such as (for example) a pressure sensor, thermal sensor (e.g., for measurement of cardiac output via thermodilution), and a fiber optics (e.g., photometric or other optical measurements). The pulmonary artery catheter can also comprise a balloon near the distal end for performing the wedge method within the pulmonary artery. The pulmonary artery catheter can be utilized in conjunction with a hemodynamic monitoring system to measure blood pressure, cardiac output, and various other hemodynamic measurements in real time. The monitoring system can further comprise one or more computational programs to assist in monitoring hemodynamic parameters and/or performing various measurements (e.g., PCWP). To perform the computational tasks, the monitoring system can include a processor, memory, display, one or more computational programs stored in the memory and run by the processor, and one or more ports for connecting the various sensors with the system.

Method 400 guides (402) the pulmonary artery catheter to the pulmonary artery. Any appropriate method to reach the pulmonary artery can be utilized. A catheter can be inserted into a central vein and navigated to the heart right atrium, then through the tricuspid valve and right ventricle, then through the pulmonary valve and into the pulmonary artery (e.g., see Figs. 2A to 2D). To assist in guidance and positioning, the pressure waveform can be monitored as the catheter is floated to the pulmonary artery. In some implementations, the catheter can comprise markings (e.g., fluoroscopic, radiographic, or sonographic) at particular distances along the proximal-distal axis and a visualization method can be utilized such as (for example) fluoroscopy, X-ray or sonography.

Once in the pulmonary artery catheter reaches the pulmonary artery, a pressure sensor can measure (404) the pulmonary artery blood pressure. In some implementation, the blood pressure sensor comprises a distal lumen in connection with a pressure transducer for measuring the blood pressure. The blood pressure measurements can be displayed on and/or recorded by the monitor system.

Method 400 further acquires (406) a PCWP measurement. In many implementations, a balloon at or near the distal end of the catheter is inflated to allow the catheter to float into the wedge position. Once the catheter is in the wedge position, the distal lumen in connection with the pressure transducer measure the blood pressure at the wedge position, which can be displayed on the monitor. As the blood pressure is measured with the pressure sensor in the wedge position, a PCWP measurement is obtained. As is understood the art, the timing of PCWP measurement is obtained in accordance with the patient's breathing and may further depend on whether the patient is spontaneously breathing or breathing via a mechanical ventilator. Generally, PCWP can be obtained at the end of a respiration cycle (i.e., upon completion of expiration). In some implementations, the PCWP measurement is displayed on a display screen of the monitoring system.

Method 400 can assess the quality of the PCWP measurement. The reliability of a PCWP measurement is dependent on the conditions of the acquisition. For example, if the distal lumen is not in the correct position or if the balloon is overinflated, the PCWP measurement can be inaccurate. Quality can be assessed by analysis and comparison of pressure waveforms within the pulmonary artery and in the wedge position.

In some implementations, a quality rating can be generated. In some implementations, the quality rating is generated via a computational process performed by the hemodynamic monitoring system. Quality ratings can be quantitative (e.g., score on a scale of 0-100) or categorical (e.g., "good" versus "bad," "adequate" versus "poor," etc.). In some implementations, thresholds are utilized to determine whether a quality score is low/high or "bad/good". Thresholds can be based on clinical data. In some implementations, a trained computational model is utilized to determine a quality rating. A machine-learning computational model can be trained utilizing clinical data. In some implementations, the quality rating is displayed on a display screen of the hemodynamic monitoring system. In some implementations, the quality rating is saved in a memory or transmitted to another computational device for storage or downstream analysis. In some implementations, a low or "bad" quality score is utilized to inform the clinician that repeating a PCWP measurement is recommended. In some implementations, a low or "bad" quality score is utilized to automatically repeat a PCWP measurement.

While a specific example of a method for performing a PCWP measurement is described above with reference to Fig. 4A, one of ordinary skill in the art can appreciate that various steps of the method can be performed in different orders and that certain steps may be optional according to various implementations. As such, it should be clear that the various steps of the method could be used as appropriate to the requirements of specific applications. Furthermore, any of a variety of methods for performing a PCWP measurement appropriate to the requirements of a given application can be utilized in various implementations.

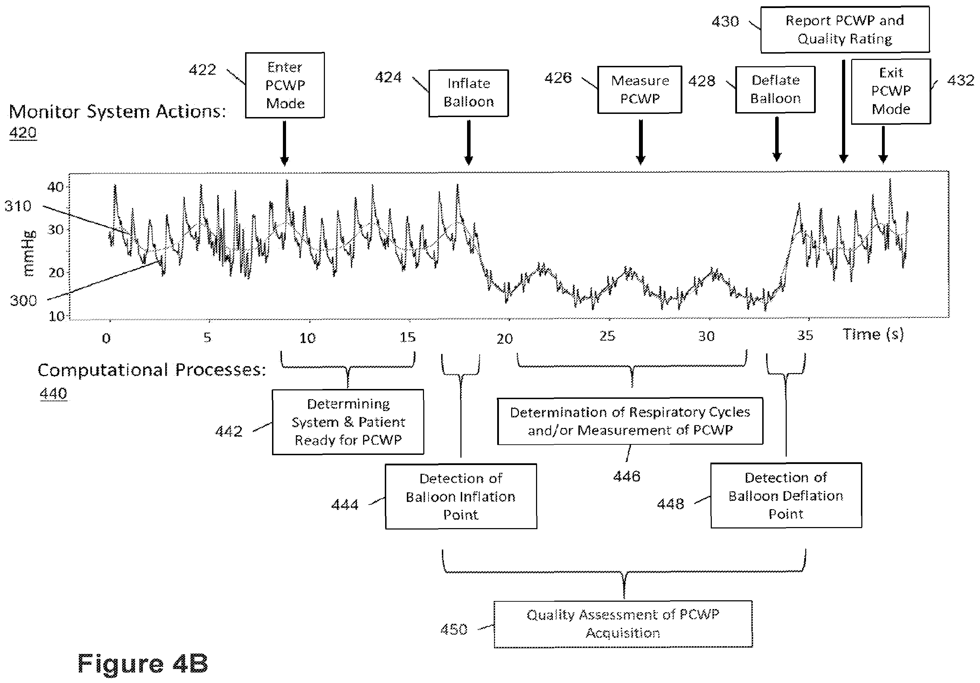

Provided in Fig. 4B is an example of a set of actions and processes that a hemodynamic monitoring system can perform to acquire a PCWP measurement and asses its quality. A hemodynamic monitoring system can perform one or more of the set of actions and processes, in any combination thereof. In some implementations, the set of actions and processes performed by the hemodynamic monitoring system enable automated acquisition and/or assessment of a PCWP measurement.

A hemodynamic monitoring system can comprise a pulmonary artery catheter (e.g., Swan-Ganz catheter) and computational processing system. The pulmonary artery catheter can comprise one or more sensors such as (for example) a pressure sensor, thermal sensor (e.g., for measurement of cardiac output via thermodilution), and a fiber optics (e.g., photometric or other optical measurements). The pulmonary artery catheter can also comprise a balloon near the distal end for performing the wedge method within the pulmonary artery. Utilizing the pulmonary artery catheter, the hemodynamic monitoring system can measure blood pressure, cardiac output, and various other hemodynamic measurements in real time. The monitoring system can further comprise one or more computational programs to assist in monitoring hemodynamic parameters and/or performing various measurements (e.g., PCWP). To perform the computational tasks, the monitoring system can include a processor, memory, display, one or more computational programs stored in the memory and run by the processor, and one or more ports for connecting the various sensors with the system.

As depicted in Fig. 4B, a hemodynamic monitoring system can perform a set of actions (420) and a set of computational processes (440). A pulmonary artery catheter can be positioned within the pulmonary artery and acquiring pressure waveform (300). To measure PCWP, the monitoring system can enter PCWP Mode (422) to initiate the set of actions and computational processes. Upon entering PCWP Mode, the monitoring system can determine whether the system and patient are ready PCWP (422). The determination of readiness can comprise a computational process to determine whether there are issues with the machinery or the current readings. In some implementations, a computational process analyzes waveform data for artifacts. The computational process can also run a set of checks, such as (for example) ensuring the pulmonary catheter and machine are properly connected and whether the patient respiration is regular. Upon determining the system and patient are ready for PCWP measurement, in some implementations, the monitoring system can provide a signal to the clinician that the balloon inflation can commence. In some implementations, upon a ready determination, the monitoring system automatically begins the process of balloon inflation.

The hemodynamic monitoring system inflates the balloon (424) at the distal end of the catheter, allowing the catheter to float to the wedge position. By assessing hemodynamic parameters derived from the pressure waveform, the hemodynamic system can detect the balloon inflation point (444), giving an indication that the catheter is wedge position. In some implementations, a computational process detects balloon inflation by a probabilistic model. In some implementations, a computational process detects balloon inflation using fuzzy logic or Boolean logic.

Upon detection of balloon inflation, in some implementations, the hemodynamic monitoring system can perform one or more computational processes to determine respiratory cycles (446). Respiratory cycles (310) are shown along with the blood pressure waveform (300) in Fig. 4B. In some implementations, respiratory cycles are extracted utilizing the blood pressure waveform. In some implementations, respiratory cycles are detected utilizing a sensor or other device in conjunction with the hemodynamic monitor. A number of techniques are known to measure respiratory rate, such as (for example) measuring airflow (e.g., flowmeter), detecting respiratory sounds (e.g., microphone), measuring air temperature (e.g., thermistor), measuring air humidity (e.g., capacitive sensor), measuring CO₂ (e.g., infrared sensor), measuring chest wall movements (e.g., accelerometer), and cardiac activity (e.g., ECG sensor). In some implementations when a mechanical ventilator is used, respiratory rate is determined by the ventilator settings.

The hemodynamic monitoring system can measure PCWP (426) utilizing a computational process for measurement of PCWP (446). In some implementations, PCWP is measured in accordance with the respiratory cycle. In certain implementations, PCWP is the pressure measured upon completion of a respiratory cycle (i.e., an end-expiratory pressure or an average of end-expiratory pressure). In some implementations, PCWP is the average pressure measured for a number of respiratory cycles during wedging (e.g., 1, 2, 3, or 4 respiratory cycles). In some implementations, PCWP is the average pressure measured for a period of time during wedging (e.g., between 2 and 20 seconds).

Upon completion of the PCWP measurement, the hemodynamic monitoring system deflates the balloon (428) at the distal end of the catheter, allowing the catheter retract back into the pulmonary artery. In a similar manner to detecting balloon inflation, the monitoring system can include a computational process for detection of the balloon deflation point (448). Accordingly, by assessing hemodynamic parameters derived from the pressure waveform, the hemodynamic system can detect the balloon deflation point, giving an indication that the catheter has retracted from the wedge position. In some implementations, a computational process detects balloon deflation by a probabilistic model. In some implementations, a computational process detects balloon deflation using fuzzy logic or Boolean logic.

The hemodynamic monitoring system can perform a quality assessment of PCWP acquisition (450) by analyzing hemodynamic data between the balloon inflation point and the balloon deflation point. In some implementations, quality of PCWP acquisition can be performed by analyzing the pressure waveform. In certain implementations, quality of PCWP acquisition can be performed by comparing the acquired PAP with the acquired PCWP. In some implementations, a trained machine-learning model is utilized to determine the quality of PCWP acquisition can be performed by analyzing. Various machine-learning models can be used, including (but not limited to) regression models, logistic regression models, neural networks, support vector machines, decision trees, adaboost, random forests, ensemble learning models (combining one or more models), and/or any other machine-learning model capable of determining a quality of a pressure reading based from feature data and classified measurements. A machine-learning model can be trained utilizing clinical data of PCWP acquisition in which high quality PCWP acquisition data is utilized to differentiate from low quality PCWP acquisition data utilizing a set of hemodynamic data features. In some implementations, the hemodynamic data features are extracted from PAP and wedge pressure waveforms. Quality ratings can be quantitative (e.g., score on a scale of 0-100) or categorical (e.g., "good" versus "bad," "adequate" versus "poor," etc.). In some implementations, thresholds are utilized to determine whether a quality score is low/high or "bad/good". Thresholds can be based on clinical data.

The hemodynamic monitoring system can report the acquired PCWP and/or the quality rating of the PCWP measurement. In some implementations, the acquired PCWP and/or the quality of the PCWP measurement is displayed on a display screen of the monitoring system. In some implementations, the quality rating is saved in a memory or transmitted to another computational device for storage or downstream analysis. In some implementations, a low or "bad" quality score is utilized to inform the clinician that repeating a PCWP measurement is recommended. In some implementations, a low or "bad" quality score is utilized to automatically repeat a PCWP measurement. Upon completion of the PCWP measurement (or a repeat of the PCWP measurement), the monitoring system can exit PCWP mode (432).

While a specific example of a set of hemodynamic monitoring system actions and computational processes for performing a PCWP measurement is described above with reference to Fig. 4B, one of ordinary skill in the art can appreciate that various actions and processes can be performed in different orders and that certain actions and processes may be optional or omitted according to various implementations. As such, it should be clear that the various actions and processes could be used as appropriate to the requirements of specific applications. Furthermore, any of a variety of actions and processes for performing a PCWP measurement appropriate to the requirements of a given application can be utilized in various implementations.

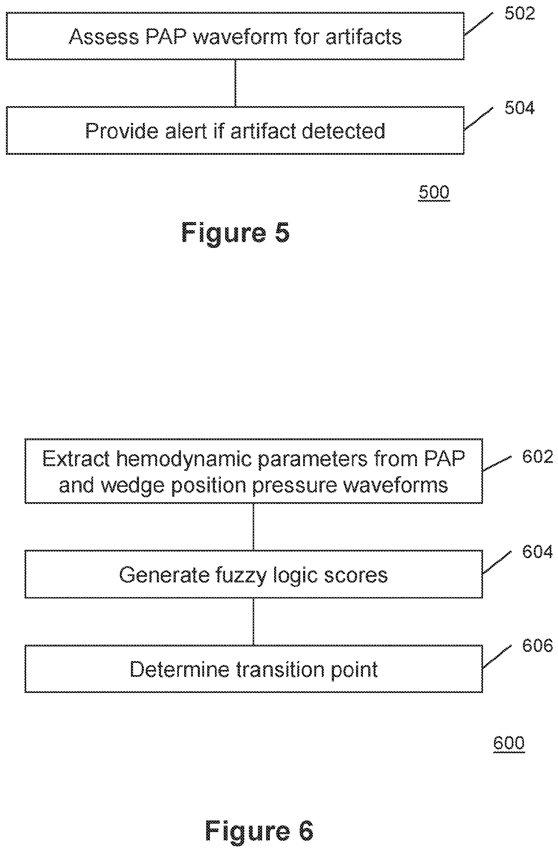

Provided in Fig. 5 is an example of a computational method to assess a pulmonary artery pressure (PAP) waveform for artifacts and alerting if detected, which can be performed utilizing a hemodynamic monitoring system. Prior to beginning a procedure to acquire PCWP measurement, a determination of whether the system and patient are in proper condition to perform the procedure can be performed. This determination can prevent poor PCWP acquisition quality, helping to ensure that high quality measurements are acquired. In some implementations, the computational method to assess a pulmonary artery pressure (PAP) waveform for artifacts is utilized as or within a computational process for determining whether the monitoring system and patient are ready for PCWP acquisition (e.g., see 442 of Fig. 4B).

Computational method 500 can be performed in real time while a PAP waveform is acquired utilizing a pulmonary artery catheter (e.g., Swan-Ganz catheter) and prior to inflation of the balloon to perform the PCWP measurement. Method 500 can assess (502) the PAP for artifacts. Artifacts can be caused by things like patient movement, measurements obtained during catheter flushing, and measurements indicating a flat line, measurements indicating underdamped, measurements indicating overdamped, and/or any other phenomenon that can result with aberrant or unnatural readings in a blood pressure waveform.

Assessment of PAP for artifacts can be performed for a period of time. In some implementations, an assessment of PAP for artifacts is performed for a period of time between 1 second and 20 seconds. In some implementations, an assessment of PAP for artifacts is performed for about a period of 1 second, about a period of 2 seconds, about a period of 3 seconds, about a period of 4 seconds, about a period of 5 seconds, about a period of 10 seconds, about a period of 15 seconds, or about a period of 20 seconds. The period of time can be further broken into smaller discrete or overlapping intervals for batch analysis.

In some implementations, a heuristic metrics can be utilized to determine if an artifact exists within the PAP waveform. In some implementations, an artifact based on flushing, improper zero point, or patient movement can be detected if the PAP maximum (*PAPₘₐₓ*) is greater than a threshold, if the PAP minimum (*PAPₘᵢₙ*) is less than threshold, and/or if the PAP minimum subtracted from the PAP maximum (*PAPₘₐₓ - PAPₘᵢₙ*) is greater than threshold. In various implementations, an artifact is detected when *PAPₘₐₓ* is greater than 60 mmHg, is greater than 80 mmHg, is greater than 100 mmHg, or is greater than 120 mmHg. In various implementations, an artifact is detected when *PAPₘᵢₙ* is less than 5 mmHg, is less than 0 mmHg, is less than -10 mmHg, is less than -20 mmHg, or is less than -30 mmHg. In various implementations, an artifact is detected when *PAPₘₐₓ - PAPₘᵢₙ* is greater than 60 mmHg, is greater than 80 mmHg, is greater than 100 mmHg, or is greater than 120 mmHg.

In some implementations, an artifact based on a flat line or overdamping can be detected if the PAP minimum subtracted from the PAP maximum (*PAPₘₐₓ - PAPₘᵢₙ*) is less than threshold. In various implementations, an artifact is detected when *PAPₘₐₓ - PAPₘᵢₙ* is less than 5 mmHg, is less than 2 mmHg, is less than 1 mmHg, or is less than 0.5 mmHg.

Upon determining an artifact exists, method 500 can provide (504) an alert. In some implementations, the hemodynamic monitoring system displays the alert on a display screen. In some implementations, when an artifact is detected, the monitoring system prevents the performance of a PCWP procedure.

While a specific example of a computational method for assessing a PAP waveform for artifacts is described above with reference to Fig. 5, one of ordinary skill in the art can appreciate that various steps of the method can be performed in different orders and that certain steps may be optional according to various implementations. As such, it should be clear that the various steps of the method could be used as appropriate to the requirements of specific applications. Furthermore, any of a variety of methods for assessing a PAP waveform for artifacts appropriate to the requirements of a given application can be utilized in various implementations.

Provided in Fig. 6 is an example of a computational method to determine transition to and/or from the wedge position, which can be performed utilizing a hemodynamic monitoring system. In order acquire a PCWP measurement, it can be beneficial to establish that the pulmonary artery catheter has transferred from the pulmonary artery to the wedge position (i.e., detection of balloon inflation point). Likewise, upon acquisition of a PCWP measurement, it can be beneficial to establish that the pulmonary artery catheter has reverted back to the pulmonary artery from the wedge position (i.e., detection of balloon deflation point). This determination can help ensure that PCWP measurement is acquired at an appropriate timepoint with the catheter in the appropriate wedge position. The determination of transition can further be utilized in the quality assessment of PCWP acquisition. In some implementations, the computational method to determine transition to and/or from the wedge position is utilized as or within a computational process for detection of the balloon inflation point and/or the balloon deflation point (e.g., see 444 and 448 of Fig. 4B).

Computational method 600 can be performed in real time upon a decision to perform a PCWP measurement acquisition and/or upon completion of a PCWP measurement acquisition. The method can help determine whether the pulmonary artery catheter properly inflated the balloon and reached the wedge position and/or help determine whether the pulmonary artery catheter properly deflated the balloon and reverted from the wedge position. Method 600 can extract (602) hemodynamic parameters from PAP and wedge position waveforms. The PAP and wedge position waveforms can be obtained from a pulmonary artery catheter (e.g., Swan-Ganz catheter).

Any hemodynamic parameters that are able discriminate between a PAP waveform and wedge position waveform can be utilized. Examples of hemodynamic parameters that can be utilized include (but are not limited to) blood pressure parameters, phase parameters, morphological features, determinable components, demographic features, and combinations thereof. Examples of blood pressure parameters that can be utilized include (but are not limited to) pulse pressure *(systolic - diastolic),* mean pressure, median pressure, diastolic pressure, systolic pressure, and/or any other obtainable blood pressure parameter. Examples of phase parameters that can be utilized include (but are not limited to) contractility, pulmonary artery compliance, stroke volume, vascular tone, afterload, full cardiac cycle, and/or any other phase parameter. Morphological features that can be utilized include (but are not limited to) a frequency component (fft), skewness, kurtosis, and/or any other obtainable morphological feature. Furthermore, determinable components include numerical analysis of waveforms, such as percentiles (e.g., 10%, 25%, 50%, 75%, 90% of systolic pressure or any other hemodynamic parameter), number of peaks above a percentile, number of valleys below a percentile, a statistical moment, histogram of data, Shannon Entropy, number of mean crossings, area under curve, singular vector coefficient after principle components analysis, a subsampled signal, and/or any other determinable component. Demographic features that can be utilized include (but are not limited to) age, gender, sex, medical history, body mass index, any other relevant features, and/or any other demographic feature. In certain implementations, the hemodynamic parameters extracted from the PAP waveform and the wedge position waveform comprise pulse pressure *(systolic - diastolic)* and mean pressure.

Utilizing the extracted hemodynamic parameters, a fuzzy logic membership function is utilized to generate (604) fuzzy logic scores to determine the state of the current pressure measurement. To generate fuzzy logic values, the difference of the extracted hemodynamic parameters of current pressure measurement to past PAP samples is computed. The difference is utilized within the fuzzy logic membership function to determine the current state.

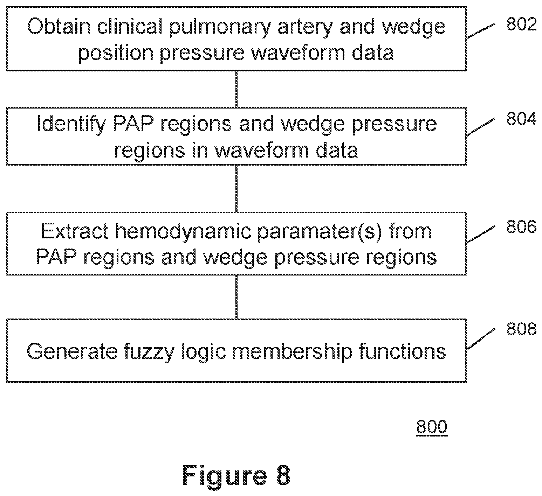

Any fuzzy logic membership function can be utilized that is appropriate to assigning membership based on the difference between the extracted hemodynamic parameters of the wedge position pressure and PAP. Examples of fuzzy logic membership functions that can be utilized include (but are not limited to) triangular, Gaussian, and trapezoidal. Further, any method to generate a fuzzy logic membership function can be utilized that is appropriate to assigning membership based on the difference between the extracted hemodynamic parameters of the wedge position pressure and PAP. Generally, clinical data of wedge position pressure and PAP waveforms can be utilized to identify hemodynamic parameters that provide robust membership classification. See Fig. 8 and associated description for an example to generate a fuzzy logic function.

In some implementations, hemodynamic parameter thresholds are utilized in conjunction with the fuzzy logic membership function to assign membership. Accordingly, in addition to assigning fuzzy logic membership, the extracted hemodynamic parameter must also be within the threshold to be assigned to particular state (e.g., transitioned to wedge position pressure or reverted back from wedge position pressure). For example, the transition to the wedge position (i.e., balloon inflation point) can be detected using a fuzzy logic membership function based on pulse pressure and mean pressure in conjunction with a mean pressure threshold (e.g., mean pressure is below threshold) and a pulse pressure threshold (e.g., pulse pressure is below threshold). In another example, the transition reverting back from the wedge position (i.e., balloon deflation point) can be detected using a fuzzy logic membership function based on pulse pressure and mean pressure or a mean pressure threshold (e.g., mean pressure is above threshold) or a pulse pressure threshold (e.g., pulse pressure is above threshold).

Based on the results of the fuzzy logic membership function (and hemodynamic parameter thresholds if utilized), method 600 determines (606) the transition point from the pulmonary artery to the wedge position (i.e., balloon inflation point) and/or reverting from the wedge position back to the pulmonary artery (i.e., balloon deflation point).

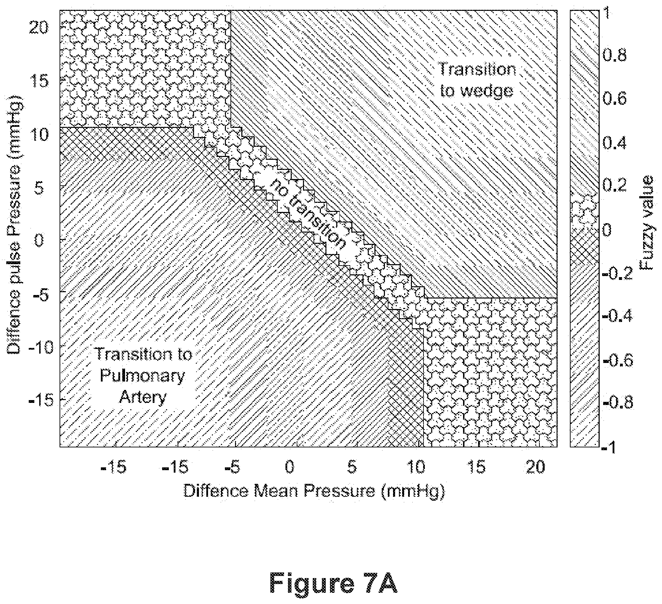

Provided in Fig. 7A is an example of fuzzy logic membership plot for plotting the transition to the wedge position and transition back to pulmonary artery in which the hemodynamic parameters utilized to discriminate the positions are pulse pressure and mean pressure. As can be seen in the membership plot, the difference of pulse pressure is plotted against the difference in mean pressure. When a computed fuzzy logic value falls within a certain region, a membership to that state can be assigned.

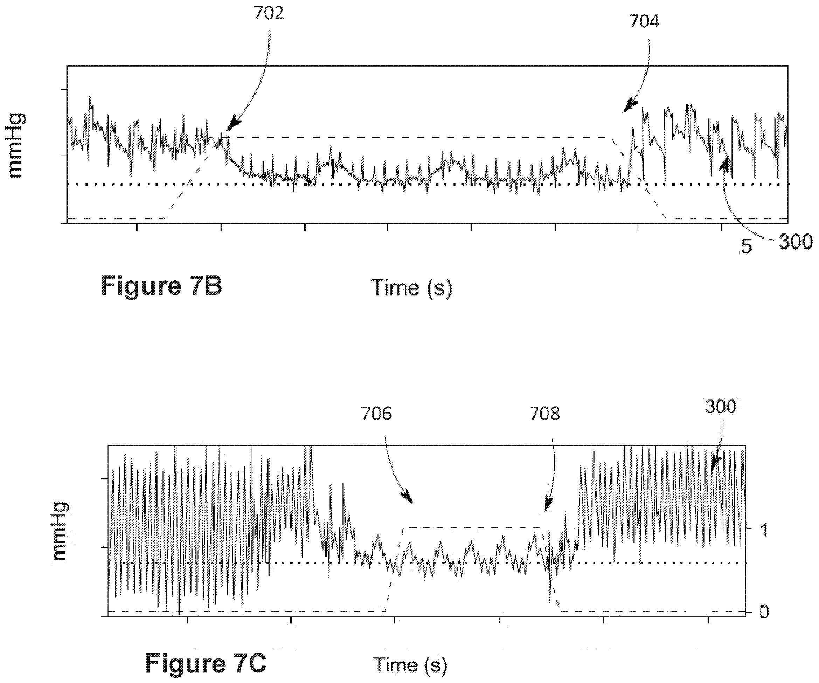

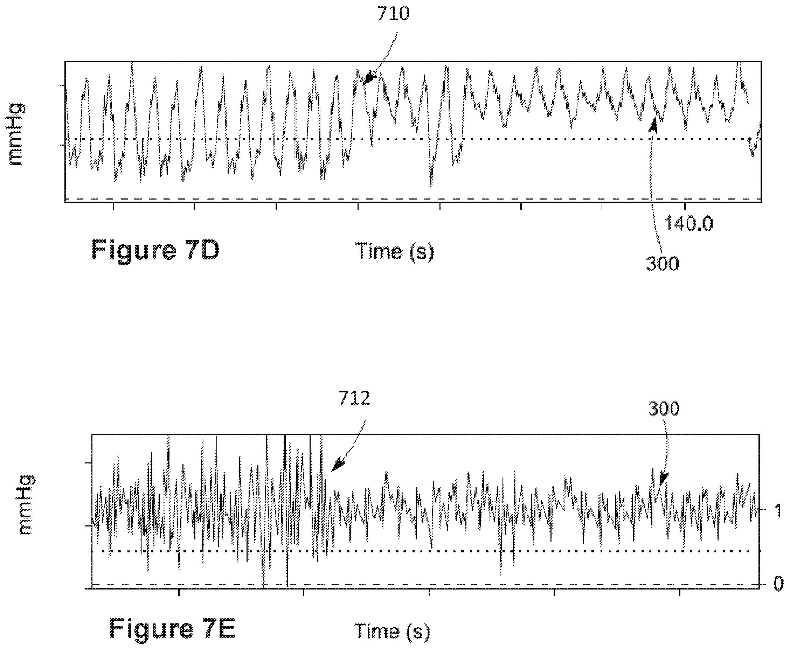

Provided in Figs. 7B to 7E are fuzzy logic membership trapezoidal functions plotted on top of pulmonary artery pressure waveforms 300 captured by a pulmonary artery catheter. The fuzzy logic membership trapezoidal functions are based on inputs of the differences of pulse pressure and mean pressure. Figs. 7B and 7C each depict a successful transition to and from the wedge position, which are marked by transitions 702, 704, 706 and 708. Figs. 7D and 7E each depict an unsuccessful transition to and from the wedge position, which are marked by transitions 710 and 712.

While a specific example of a computational method for determining transition to and/or from a wedge position is described above with reference to Fig. 6, one of ordinary skill in the art can appreciate that various steps of the method can be performed in different orders and that certain steps may be optional according to various implementations. As such, it should be clear that the various steps of the method could be used as appropriate to the requirements of specific applications. Furthermore, any of a variety of methods for determining transition to and/or from a wedge position appropriate to the requirements of a given application can be utilized in various implementations.

Provided in Fig. 8 is an example of a computational method for generating fuzzy logic membership functions for determining transition to and/or from a wedge position. The generated fuzzy logic functions can be utilized for real time assessment of transition of a pulmonary artery catheter (e.g., Swan-Ganz catheter) from the pulmonary artery to the wedge position (i.e., balloon inflation point) and/or reverting from the wedge position back to the pulmonary artery (i.e., balloon deflation point).

Computational method 800 obtains (802) clinical pulmonary artery and wedge position pressure waveform data, which can be obtained from PCWP procedures. The waveform data should have discernible transitions between the pulmonary artery and the wedge position.

Method 800 identifies (804) PAP regions and wedge pressure regions in waveform data. Generally, the selected regions should be clearly within the PAP state or clearly within the wedge pressure state.

Method 800 extracts one or more hemodynamic parameters from the PAP regions and the wedge pressure regions. Any hemodynamic parameters that are able discriminate between a PAP waveform and wedge position waveform can be utilized. Examples of hemodynamic parameters that can be utilized include (but are not limited to) blood pressure parameters, phase parameters, morphological features, determinable components, demographic features, and combinations thereof. Examples of blood pressure parameters that can be utilized include (but are not limited to) pulse pressure *(systolic - diastolic),* mean pressure, median pressure, diastolic pressure, systolic pressure, and/or any other obtainable blood pressure parameter. Examples of phase parameters that can be utilized include (but are not limited to) contractility, pulmonary artery compliance, stroke volume, vascular tone, afterload, full cardiac cycle, and/or any other phase parameter. Morphological features that can be utilized include (but are not limited to) a frequency component (fft), skewness, kurtosis, and/or any other obtainable morphological feature. Furthermore, determinable components include numerical analysis of waveforms, such as percentiles (e.g., 10%, 25%, 50%, 75%, 90% of systolic pressure or any other hemodynamic parameter), number of peaks above a percentile, number of valleys below a percentile, a statistical moment, histogram of data, Shannon Entropy, number of mean crossings, area under curve, singular vector coefficient after principle components analysis, a subsampled signal, and/or any other determinable component. Demographic features that can be utilized include (but are not limited to) age, gender, sex, medical history, body mass index, any other relevant features, and/or any other demographic feature. In certain implementations, the hemodynamic parameters extracted from the PAP waveform and the wedge position waveform comprise pulse pressure *(systolic - diastolic)* and mean pressure.

Parameters for generated fuzzy logic memberships can be selected for various purpose. Parameters can be selected computing power, ease of measuring, correlation, accuracy, any other relevant parameter and/or metric, and combinations thereof. Correlation can be correlation to another feature, such as a correlation to pulse pressure and/or any other feature.

Method 800 generates fuzzy logic membership functions using one or more extracted hemodynamic parameters. To generate fuzzy logic membership functions, the difference of the extracted hemodynamic parameters between the wedge pressure regions and PAP regions is computed. The difference is utilized within the fuzzy logic membership function to determine whether the hemodynamic parameter provides a robust differential determination of the states.

In some implementations, hemodynamic parameter thresholds are utilized in conjunction with the fuzzy logic membership function to assign membership. Accordingly, in addition to assigning fuzzy logic membership, the extracted hemodynamic parameter must also be within the threshold to be assigned to particular state (e.g., transitioned to wedge position pressure or reverted back from wedge position pressure). For example, the transition to the wedge position (i.e., balloon inflation point) can be detected using a fuzzy logic membership function based on pulse pressure and mean pressure in conjunction with a mean pressure threshold (e.g., mean pressure is below threshold) and a pulse pressure threshold (e.g., pulse pressure is below threshold). In another example, the transition reverting back from the wedge position (i.e., balloon deflation point) can be detected using a fuzzy logic membership function based on pulse pressure and mean pressure or a mean pressure threshold (e.g., mean pressure is above threshold) or a pulse pressure threshold (e.g., pulse pressure is above threshold).

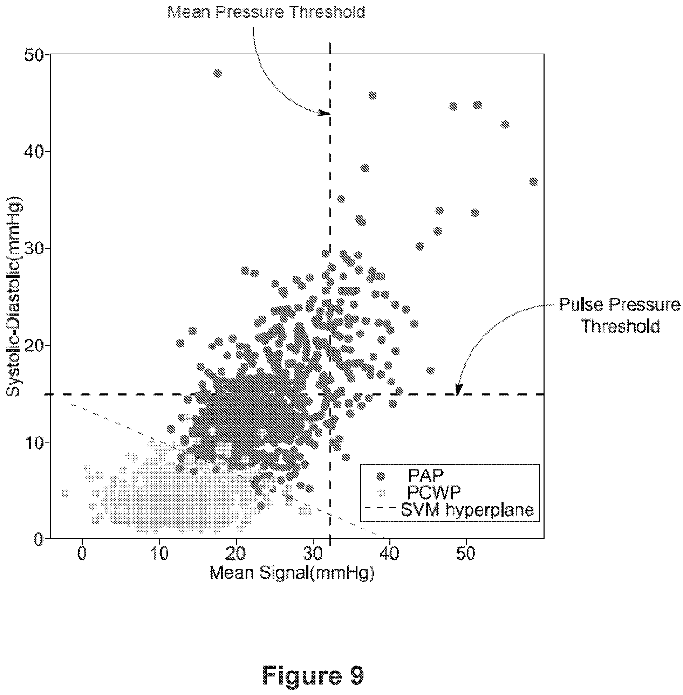

Provided in Fig. 9 is a plot of pulse pressure *(systolic - diastolic)* and mean pressure (mean signal) derived from clinical pulmonary artery and wedge position pressure waveform data of a large cohort of patients. As can be seen from the plot, the hemodynamic parameter data derived from wedge position waveform resides primarily in the lower left quadrant. To ensure derived hemodynamic parameter data in fact derived from the wedge position waveform, a pulse pressure threshold (e.g., 15 mmHg) and a mean pressure threshold (e.g., 32 mmHg) can be applied.

While a specific example of a computational method for generating fuzzy logic membership functions is described above with reference to Fig. 6, one of ordinary skill in the art can appreciate that various steps of the method can be performed in different orders and that certain steps may be optional according to various implementations. As such, it should be clear that the various steps of the method could be used as appropriate to the requirements of specific applications. Furthermore, any of a variety of methods for generating fuzzy logic membership functions appropriate to the requirements of a given application can be utilized in various implementations.

Various other methodologies can be utilized in accordance with some implementations to segregate, classify, and/or otherwise analyze data to identify clusters for detecting transition to and/or from the wedge position. Such methods can include algorithmic, machine learning (e.g., artificial intelligence), statistical, and/or any other method to identify data clusters. Various machine-learning models, including (but not limited to) convolutional neural networks (CNNs), support vector machines (SVMs), and/or any other machine-learning model sufficient for identifying features to classify catheter position.

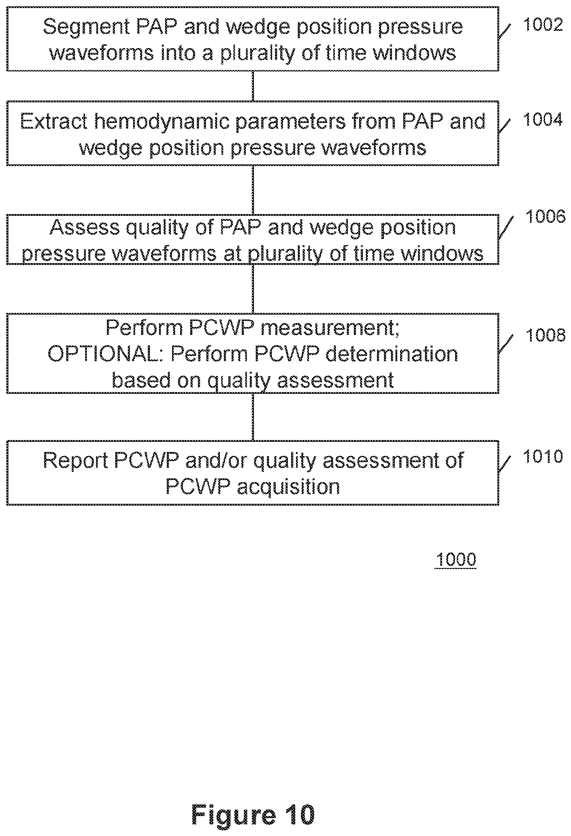

Provided in Fig. 10 is an example of a computational method to perform a PCWP measurement and assess the quality of PCWP acquisition, which can be performed utilizing a hemodynamic monitoring system. Determining the quality of PCWP acquisition can help inform a clinician whether the PCWP measurement is good to make a health determination or whether to repeat the PCWP procedure. Further, assessment of the quality of wedge position pressure acquisition can be utilized to improve PCWP computation by only utilizing portions of the wedge position pressure waveform that are above a quality standard. Quality of PCWP acquisition can be determined using one or more trained machine-learning models and/or various hemodynamic thresholds. In some implementations, the computational method to perform a and assess the quality of PCWP acquisition can be combined with a set of other computational processes to perform a high-quality, automated PCWP measurement (e.g., see 450 of Fig. 4B).

Computational method 1000 can be performed in real time to improve and/or assess the quality of PCWP measurement acquisition. Method 1000 segments (1002) PAP and wedge position waveforms into a plurality of temporal windows. The PAP and wedge position waveforms can be obtained from a pulmonary artery catheter (e.g., Swan-Ganz catheter).

The plurality of temporal windows can be discrete or overlapping, contiguous or non-contiguous. Temporal windows can be defined by any non-zero time period up to and including the full time frame of a PAP or wedge position waveform. In some implementations, a temporal window length is between 0.5 seconds and 20 seconds. In various implementations, a temporal window length is between 0.5 seconds and 1.5 seconds, between 1.0 seconds and 2.0 seconds, between 1.0 seconds and 3.0 seconds, between 2.0 seconds and 4.0 seconds, between 3.0 seconds and 5.0 seconds, between 4.0 seconds and 6.0 seconds, between 5.0 seconds and 7.0 seconds, between 6.0 seconds and 8.0 seconds, between 7.0 seconds and 9.0 seconds, between 8.0 seconds and 10.0 seconds, between 9.0 seconds and 11.0 seconds, between 10.0 seconds and 15.0 seconds, between 12.5 seconds and 17.5 seconds, or between 15.0 seconds and 20.0 seconds. In some implementations, temporal windows are defined by a physiological event (e.g. one or more respiration cycles). In some implementations, a user (e.g., clinician) can select a temporal window length.

Method 1000 extracts one or more hemodynamic parameters from the PAP regions and the wedge pressure regions. Any hemodynamic parameters that are able discriminate between a PAP waveform and wedge position waveform can be utilized. Examples of hemodynamic parameters that can be utilized include (but are not limited to) blood pressure parameters, phase parameters, morphological features, determinable components, demographic features, and combinations thereof. Examples of blood pressure parameters that can be utilized include (but are not limited to) pulse pressure (*systolic - diastolic*), mean pressure, median pressure, diastolic pressure, systolic pressure, and/or any other obtainable blood pressure parameter. Examples of phase parameters that can be utilized include (but are not limited to) contractility, pulmonary artery compliance, stroke volume, vascular tone, afterload, full cardiac cycle, and/or any other phase parameter. Morphological features that can be utilized include (but are not limited to) a frequency component (fft), skewness, kurtosis, and/or any other obtainable morphological feature. Furthermore, determinable components include numerical analysis of waveforms, such as percentiles (e.g., 10%, 25%, 50%, 75%, 90% of systolic pressure or any other hemodynamic parameter), number of peaks above a percentile, number of valleys below a percentile, a statistical moment, histogram of data, Shannon Entropy, number of mean crossings, area under curve, singular vector coefficient after principle components analysis, a subsampled signal, and/or any other determinable component. Demographic features that can be utilized include (but are not limited to) age, gender, sex, medical history, body mass index, any other relevant features, and/or any other demographic feature. In certain implementations, the hemodynamic parameters extracted from the PAP waveform and the wedge position waveform comprise pulse pressure (*systolic - diastolic*) and mean pressure.

Method 1000 assesses quality of PAP and wedge position pressure waveforms at a plurality of the segmented time windows. Quality can be assessed by various methodologies, which may be combined. In some implementations, quality is assessed utilizing a trained machine-learning model. In some implementations, quality is assessed utilizing a threshold for various hemodynamic parameters. Quality ratings can be quantitative (e.g., score on a scale of 0-100) or categorical (e.g., "good" versus "bad," "adequate" versus "poor," etc.). In some implementations, thresholds are utilized to determine whether a quality score is low/high or "bad/good". Thresholds can be based on clinical data.

To assess quality of PAP and wedge position pressure waveforms utilizing a machine-learning model, one or more parameters are entered to the model as features to yield an assessment of quality. Various machine-learning models can be used, including (but not limited to) regression models, logistic regression models, neural networks, convolutional neural networks, support vector machines, decision trees, adaboost, random forests, ensemble learning models (combining one or more models), and/or any other machine-learning model capable of determining a quality of a pressure reading based from feature data and classified measurements. A machine-learning model can be trained utilizing clinical data of PAP and wedge position pressure acquired in which high quality time windows of data is utilized to differentiate from low quality time windows of data utilizing a set of hemodynamic data features. In some implementations, the hemodynamic data features are extracted from the clinical PAP and wedge pressure waveforms and associated their data window. The data windows can be differentiated as derived from PAP or wedge position waveform, based on their quality, assigned a quality score, or any other quality indicator. The machine-learning model can be trained to identify the quality of PAP and wedge pressure waveform windows. In some implementations, quality is determined by the ability to different a window of a wedge pressure waveform from a window of a PAP waveform.

In some implementations, quality is assessed based on whether the window of a waveform is above or below a threshold for various hemodynamic parameters. For a number of hemodynamic parameters, PAP waveforms and wedge position waveforms are expected to be within certain ranges, as determined by clinical data. Further, some hemodynamic parameters are expected to highly differential between PAP waveforms and wedge position waveform, as determined by clinical data. By utilizing these expected ranges or expected differences between the waveforms, windows of these waveforms can be assessed for quality. In some implementations, a window of a waveform is determined to be above a quality standard if it meets one or more criteria. In some implementations, a window of a waveform is determined to be below a quality standard if it fails to meet one or more criteria.

Parameter thresholds can be based on one or more qualitative, quantitative, or semi-quantitative factors that can assess quality of a window. Non-limiting examples of thresholds that can be used for identifying parameters can be based on professional experiences and/or metrics identified in literature. Parameter thresholds that can be used (alone or in combination) as a quality metric of window include (but are not limited to) increase/decrease of pressure beyond a threshold, including (but not limited to) mean pressure and pulse pressure; mean wedge position pressure below pulmonary artery diastolic pressure; increase/decrease in blood oxygen beyond a threshold; increase of respiration induced variation in a wedge position as compared to pulmonary artery position; and/or morphological features such as clearly defined waves in pressure (e.g., "a" and "v" waves). Using one or more quality features, a quality score can be generated for windows of a waveform, which can categorical, quantitative, or semi-quantitative quality scores. Qualitative categories can be binary (e.g., good or bad) and/or a qualitative ranking (e.g., poor, middling, good).

In some implementations, multiple assessments for quality are combined, which can be treated as factors and/or requirements of quality assessment. For instance, the quality of a window can be determined using a number of weight factors, each of the factors combined to yield an overall determination of quality. Alternatively, or in addition, the quality of a window must meet all of one or more requirements to meet a standard of quality and failure to meet a any one of the one or more requirements yields failure to meet that standard. In some implementations when a machine-learning model is combined with hemodynamic one or more parameter thresholds, the thresholds can be applied as a pre-requisite for entry into the model, in an ensemble with the model, or as assessment after the model.

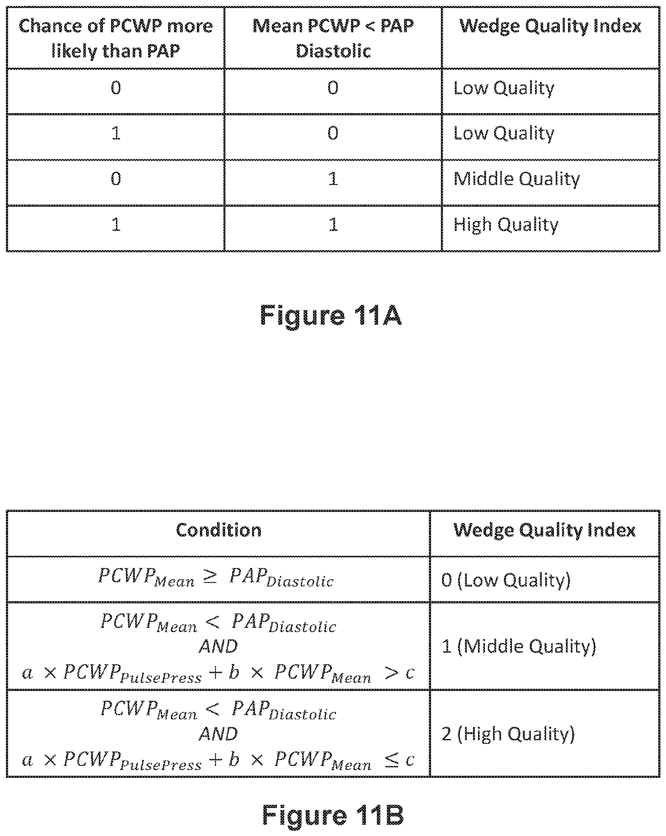

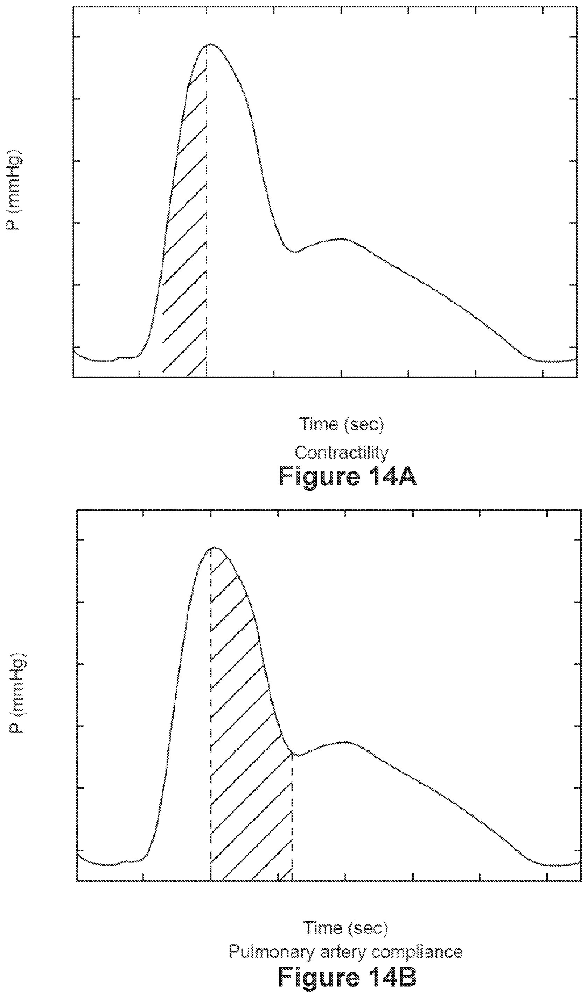

Figures 11A and 11B provide an example of assessing waveform window quality. Specifically, Figure 11A provides a table of assessments that can be made on a binary basis (e.g., true-false, 0-1, etc.) to categorize windows of a waveform. These assessments utilize a chance (or likelihood) of a PCWP over a PAP position and the mean PCWP pressure is less than the mean PAP diastolic pressure. Based on binary decisions, quality can be assessed. Similarly, Figure 14B provides multiple binary requirements based on extracted parameters, including whether mean PCWP pressure (PCWP_{Mean}) is less than the mean PAP diastolic pressure (PAP_{Diastolic}) and whether a weighted product of mean PCWP pulse pressure (PCWP_{PulsePress}) and mean PCWP pressure (PCWP_{Mean}) is greater than a determinant value or less than or equal to the determinant value. Although specific equations are depicted in Fig. 11B, various equation can be generated or altered depending on specific parameters utilized in the equation. Further, any equation for generating a hyperplane, where weights a and b and determinant value c can be revealed as an equation describing the hyperplane..

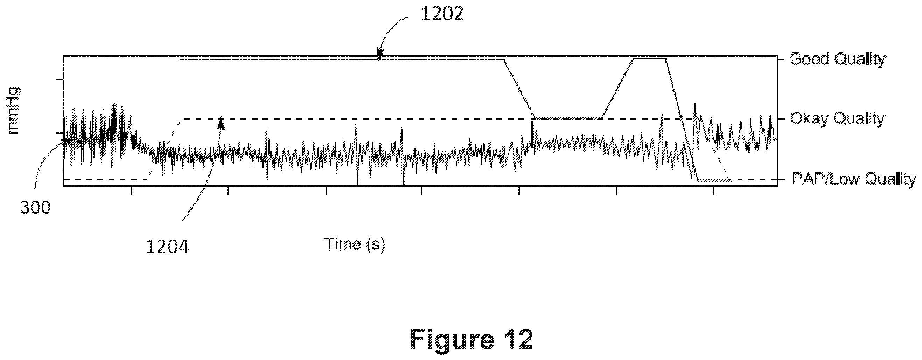

Figure 12 provides an assessment of quality 1202 of windows of waveform 300 overlaid on top of the waveform. The wedge position portion 1204 of waveform 300 can be assessed a quantitative or qualitative quality score. As can be seen, a few windows of the wedge position portion 1204 are of "Okay Quality."

Method 1000 performs a PCWP measurement. In some implementations, PCWP is measured in accordance with the respiratory cycle. In certain implementations, PCWP is the pressure measured upon completion of a respiratory cycle (i.e., an end-expiratory pressure or an average of end-expiratory pressure). In some implementations, PCWP is the average pressure measured for a number of respiratory cycles during wedging (e.g., 1, 2, 3, or 4 respiratory cycles). In some implementations, PCWP is the average pressure measured for a period of time during wedging (e.g., between 2 and 20 seconds). In some implementations, a PCWP measurement is acquired and then that measurement is assessed for quality.

Quality assessments can be utilized to enhance the determination of PCWP. In some implementations, quality assessments are utilized to enhance determination of the PCWP measurement. In some implementations, the quality of a plurality of windows of wedge position waveform is determined and if a certain number of windows fail to meet a quality standard, no PCWP measurement is determined. In some implementations, only windows meeting a standard of quality are utilized to determine the PCWP measurement. In some implementations, windows that fail to meet a standard of quality are discarded prior to determining the PCWP measurement.

Method 1000 reports PCWP and/or quality assessment of PCWP acquisition. In some implementations, the acquired PCWP and/or the quality of the PCWP measurement is displayed on a display screen of the monitoring system. In some implementations, the quality rating is saved in a memory or transmitted to another computational device for storage or downstream analysis. In some implementations, a low or "bad" quality score is utilized to inform the clinician that repeating a PCWP measurement is recommended. In some implementations, a low or "bad" quality score is utilized to automatically repeat a PCWP measurement. Upon completion of the PCWP measurement (or a repeat of the PCWP measurement), the monitoring system can exit PCWP mode (432).

In determining quality, certain systems and/or methods of this disclosure are directed to identifying optimized combinations of input parameters to produce a quality rating of a pressure waveform. The goal of optimization can be any one or any combination of reducing labor, reducing cost, reducing risk, increasing reliability, increasing efficacies, reducing side effects, reducing toxicities, and alleviating drug resistance, among other benefits.

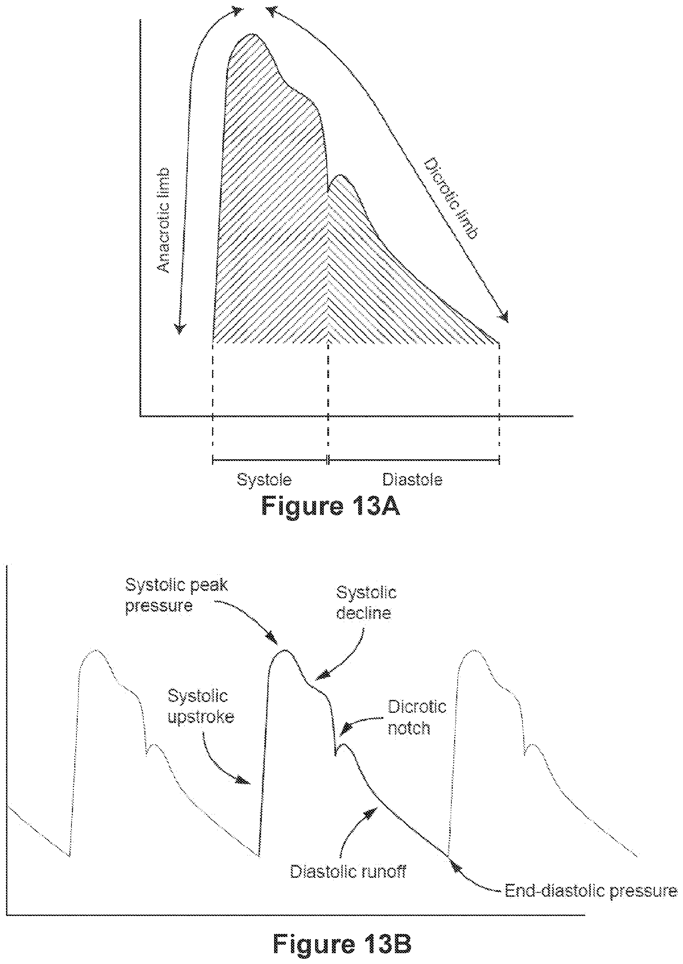

Blood pressure waveforms have various discernable characteristics that can be identified within a singular beat waveform (e.g., a single heartbeat cycle of systole and diastole) or over several beat waveforms (e.g., several heartbeat cycles). Turning to Figures 13A and 13B, general waveform characteristics are illustrated relative to a single beat. In particular, Figure 13A illustrates portions of a waveform that represent systole and diastole as well as the anacrotic and dicrotic limbs in a single beat. Figure 13B illustrates the granular characteristics of a beat waveform, identifying the systolic upstroke, peak pressure, and decline as well as dicrotic notch, diastolic runoff, and the end diastolic pressure.

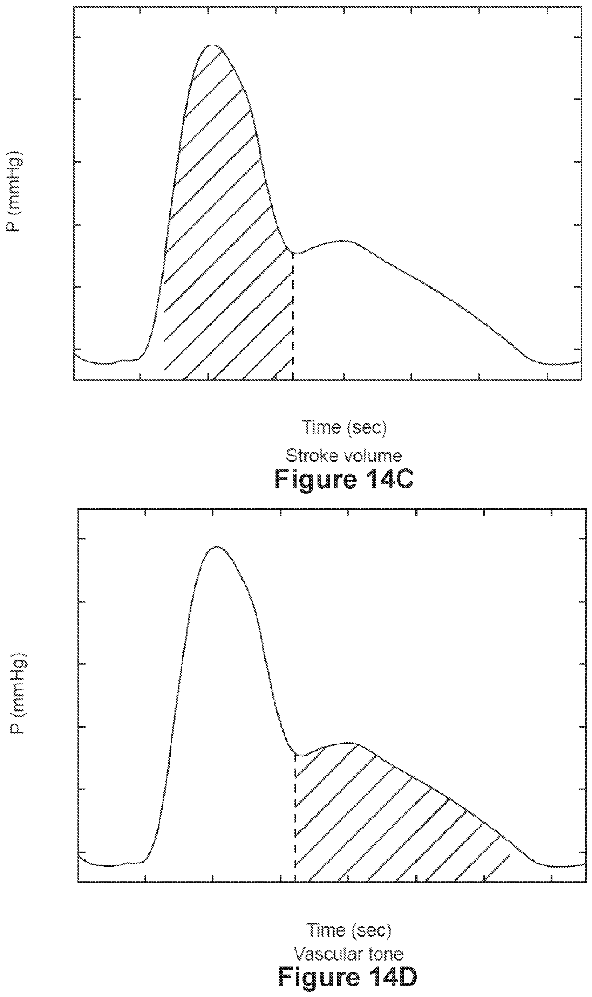

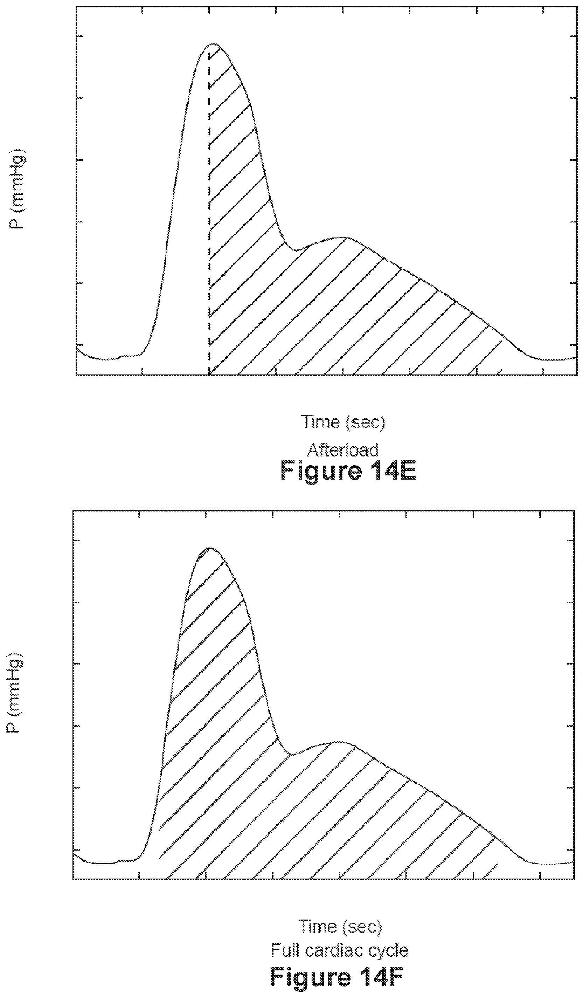

In addition to the characteristics identified in Figures 13A and 13B, a number of systems and/or methods identify and measure or calculate a variety of additional characteristics within a single beat's waveform. Turning to Figures 14A to 14F, waveform phase characteristics identified from a single beat can include one or more of contractility (Figure 14A), pulmonary artery compliance (Figure 14B), stroke volume (Figure 14C), vascular tone (Figure 14D), afterload (Figure 14E), and/or full cardiac cycle (Figure 14F). The characteristics represented in each of Figures 14A to 14F is the shaded portion of the waveform. In addition to singular measurements, various systems and/or methods determine one or more determinable characteristics. Such determinable characteristics can be determined from one or more of each phase in the pressure waveform, including (but not limited to) mean of the phase, maximum, minimum, duration, area, standard deviation, slopes, derivatives (as a difference in pressure over time (dP/dt)), trends, deviations from trends, variation, variance, and combinations thereof. Certain systems and/or methods also determine one or more morphological characteristics. Such morphological characteristics can be determined from one or more waveform phase, and such morphological features include (but are not limited to) frequency components (fft), skewness, kurtosis, any other morphological characteristic determinable within a waveform, and combinations thereof. With any one or more of the foregoing characteristics (e.g., phase characteristics, determinable characteristics, morphological characteristics, etc.), additional systems and/or methods determine one or more of complexity (e.g., entropy measure such as approximate entropy and/or sample entropy), baroreflex sensitivity (e.g., via cross correlation analysis), variability, changes, and combinations thereof.

Based on a pressure waveform, various systems and/or methods further extract one or more of the following parameters: heart rate, respiratory rate, stroke volume, pulse pressure, mean pulmonary artery pressure (mPAP), pulmonary artery systolic pressure (sPAP), pulmonary artery diastolic pressure (dPAP), pulse pressure variation, stroke volume variation, heart rate variability, cardiac output, pulmonary peripheral resistance, vascular compliance, vascular elasticity, right ventricular contractility (dP/dt).

Certain catheters (e.g., Swan-Ganz catheters) include temperature probes, fiber optics, and or other components capable of providing additional measurements. As such, certain systems and/or methods can extract characteristics such as cardiac output, stroke volume, ejection fraction, end diastolic volume, blood temperature, blood oxygenation, any other measurement capable of being obtained from the catheter, and combinations thereof. It should be noted that the foregoing additional measurements can be measured directly (e.g., a singular measurement) or extracted from a waveform. Additionally, numerous systems and/or methods extract additional characteristics from any of such additional measurement (e.g., determinable features, morphological features, etc.) as noted previously.

Various systems and methods utilize one or more of the foregoing characteristics and characteristic types (e.g., morphological characteristics, phase characteristics, etc.) to identify a wedge transition and/or quality of a pressure waveform during a PCWP procedure, such as will be described further in detail.

### Systems of hemodynamic monitoring and computation

A computational processing system to perform and assess the quality of PCWP measurements in accordance with the various methods and processes of the disclosure typically utilizes a processing system including one or more of a CPU, GPU and/or neural processing engine. As described herein, digit arterial pressure can be recorded and transformed into radial arterial pressure in real time using a computational processing system.

The computational processing system can be housed within a hemodynamic monitoring system in a direct wired connection between the monitoring and components, inclusive of a pulmonary artery catheter (e.g., Swan-Ganz catheter). Alternatively, the computational processing system can be housed separately from the hemodynamic monitoring system and components, receiving the acquired pulmonary artery pressure via a wireless connection (e.g., WiFi, cellular, Bluetooth, etc). The computational processing system can be implemented on any appropriate computing device such as (but not limited to) a hemodynamic monitoring system, a tablet and/or a portable computer.

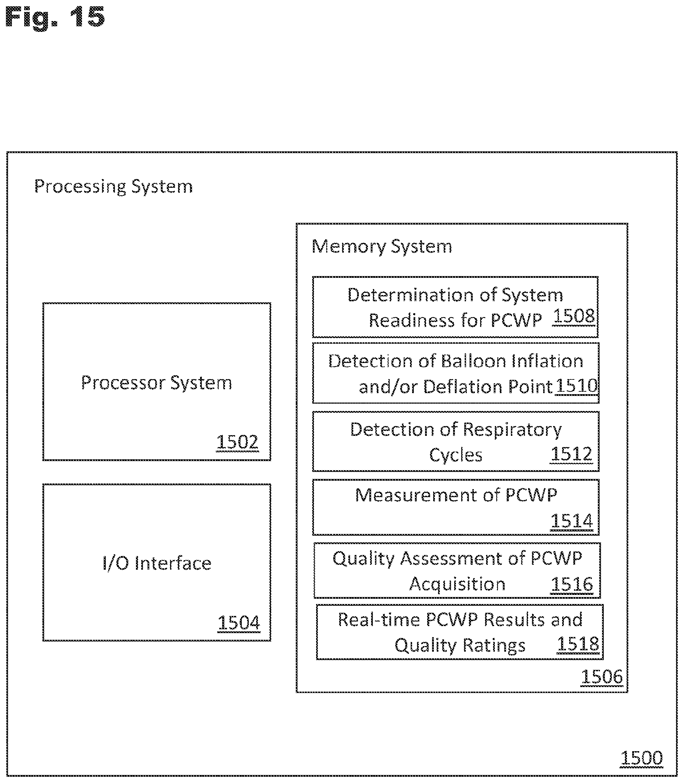

An exemplary computational processing system that can be utilized to perform the various methods and processes of the disclosure is illustrated in Fig. 15. The computational processing system 1500 includes a processor system 1502, an I/O interface 1504, and a memory system 1506. As can readily be appreciated, the processor system 1502, I/O interface 1504, and memory system 1506 can be implemented using any of a variety of components appropriate to the requirements of specific applications including (but not limited to) CPUs, GPUs, ISPs, DSPs, wireless modems (e.g., WiFi, Bluetooth modems), serial interfaces, volatile memory (e.g., DRAM) and/or non-volatile memory (e.g., SRAM, and/or NAND Flash).

In the illustrated example, the memory system is capable of storing various data, applications, and models. It is to be understood that the listed data, applications and models are a representative sample of what can be stored in memory and that various memory systems may store some or all of the various data, applications, and models listed. Further, any combination of data, applications, and models can be stored, and in some implementations, various data, applications, and/or models are stored temporarily.

In some implementations, the memory system 1506 can store (for example) one or more of the following applications: Determination of System Readiness for PCWP 1508, Detection of Balloon Inflation and/or Deflation Point 1510, Detection of Respiratory Cycles 1512, Measurement of PCWP 1514, and Quality Assessment of PCWP Acquisition 1516. The various applications can be provided as individual processes or as an ensemble of processes, each of which may be utilized to provide automatic acquisition and quality assessment of PCWP measurements. Real-time PCWP results and quality ratings 1518 can also optionally be stored on memory system 1506 and/or displayed on a display screen via the I/O interface 150404.

While specific computational processing systems are described above with reference to Fig. 15, it should be readily appreciated that computational processes and/or other processes utilized in the provision of performing and assessing the quality of PCWP measurements can be implemented on any of a variety of processing devices including combinations of processing devices. Accordingly, computational devices should be understood as not limited to specific monitoring systems, computational processing systems, and/or specific applications and models. Computational devices can be implemented using any of the combinations of systems described herein and/or modified versions of the systems described herein to perform the processes, combinations of processes, and/or modified versions of the processes described herein.

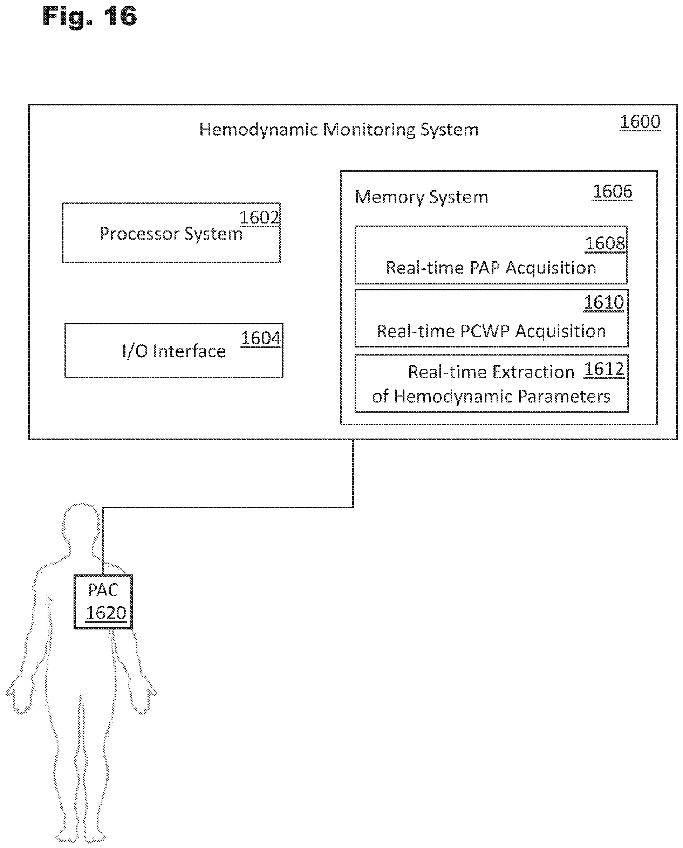

The systems and methods of the current disclosure can be utilized within a hemodynamic monitoring system. Generally, the hemodynamic monitoring system includes a pulmonary artery catheter (PAC; e.g., Swan-Ganz catheter). Provided in Fig. 16 is an example of a hemodynamic monitoring system 1600 as would be utilized to measure PCWP of an individual. Within the patient is a PAC 1620 that may comprise one or more sensors such as (for example) a pressure sensor, thermal sensor (e.g., for measurement of cardiac output via thermodilution), and a fiber optics (e.g., photometric or other optical measurements). PAC 1620 can also comprise a balloon near the distal end for performing the wedge method within the pulmonary artery. PAC 1620 can be in connection with hemodynamic monitoring system 1600. A pump system can be provided to inflate the balloon.

Hemodynamic monitoring system 1600 can comprise a computational system, such as (for example) the system portrayed and described in reference to Fig. 8. Hemodynamic monitoring system 1600 can comprise a processor system 1602 and I/O interface 1604 for input and output of data, such as data communicated between hemodynamic monitoring system 1600, a sensor of PAC 1620, and a user interface. Hemodynamic monitoring system 900 can utilize a number of applications stored within a memory system 1606 to be executed by processor system 1602. Applications that can be stored within a memory system 1606 include real-time PAP acquisition applications 1608, real-time PCWP acquisition applications 1610, and real-time extraction of hemodynamic parameters 1612 for operating the hemodynamic monitoring system.

While a specific hemodynamic monitoring system configuration is described above with reference to Fig. 16, it should be readily appreciated that various hemodynamic monitoring systems and/or other medical monitoring utilized in the provision of hemodynamic monitoring can be implemented in any of a variety of configurations. Accordingly, the various systems and methods described herein should be understood as not to be limited to any specific hemodynamic monitoring system, but instead can be implemented using any variety of hemodynamic monitoring or medical monitoring systems capable of measuring PAP and PCWP.

## Claims

1. A hemodynamic monitoring system (1600) for performing and assessing quality of a pulmonary capillary wedge pressure (PCWP) measurement, comprising:
a pulmonary artery catheter (1620) configured to acquire blood pressure measurement acquired from a pulmonary artery position and a blood pressure measurement acquired from a wedge position, wherein the pulmonary artery catheter comprises a balloon that is inflatable; and
a computational processing system in connection with the pulmonary artery catheter, the computational processing system comprising:
a processor system (1602);
a display screen in digital connection with the processor system; and
a memory system (1606) comprising one or more applications that can direct the processor system to:
acquire a blood pressure measurement in a pulmonary artery position;
inflate the balloon, wherein inflating the balloon allows the catheter to migrate to a wedge position;
acquire a blood pressure measurement in the wedge position;
generate a blood pressure waveform from blood pressure measurement acquired from a pulmonary artery position and a blood pressure measurement acquired from a wedge position;
determine a PCWP measurement from the blood pressure measurement from the wedge position;
determine a quality assessment for the PCWP measurement using a machine-learning model configured to provide a quality assessment for the PCWP measurement based on the blood pressure measurement acquired from the pulmonary artery position and the blood pressure measurement acquired from the wedge position; and
display one or more of: the blood pressure waveform, the PCWP measurement, or the quality assessment for the PCWP measurement on the display screen.

2. The system of claim 1, wherein the blood pressure waveform is generated and displayed on the display screen in real time.

3. The system of claim 1 or 2, wherein the PCWP measurement is determined and displayed on the display screen in real time.

4. The system of any preceding claim, wherein the quality assessment for the PCWP measurement is determined and displayed on the display screen in real time.

5. The system of any preceding claim, wherein the one or more applications can direct the processor system to:
prior to inflating the balloon, assess in real time the blood pressure measurement acquired from a pulmonary artery position for artifacts.

6. The system of any preceding claim, wherein the one or more applications can direct the processor system to:
detect in real time a transition from a pulmonary artery position to a wedge position or from a wedge position to a pulmonary artery position by:
extracting one or more hemodynamic features from the blood pressure measurement in the pulmonary position and the blood pressure measurement in the wedge position; and
determining, using a fuzzy logic membership function, a fuzzy logic value based on a change in value between the blood pressure measurement in the pulmonary position and the blood pressure measurement in the wedge position for each hemodynamic feature of the one or more hemodynamic features.

7. The system of claim 6, wherein the one or more features comprise mean pressure and pulse pressure.

8. The system of any preceding claim, wherein the one or more applications can direct the processor system to:
segment the blood pressure measurement acquired from the pulmonary artery position and the blood pressure measurement acquired from the wedge position into temporal windows; and
extract features from the temporal windows of the blood pressure measurement acquired from the pulmonary artery position and the blood pressure measurement acquired from the wedge position; and
enter the extracted features from the temporal windows into the machine-learning model to yield a quality assessment for each temporal window; wherein the quality assessment is based on whether the extracted features for each temporal window can be differentiated as derived from the pulmonary artery position or from the wedge position to yield the quality assessment for the PCWP, wherein the machine-learning model is trained to detect whether an extracted feature of a temporal window is derived the blood pressure measurement acquired from the pulmonary artery position or blood pressure measurement acquired from the wedge position.

9. The system of claim 8, wherein the one or more applications can direct the processor system to:
determine whether one or more extracted features of a temporal window are above or below a threshold to yield the quality assessment for the PCWP measurement.

10. The system of claim 9, wherein the one or more extracted features comprise: *PCWP_{Mean}, PAP_{Diastolic},* and *PCWP_{PulsePress}*, wherein *PCWP_{Mean}* is a mean value of pressure measurements acquired in the wedge position; wherein *PAP*_{Diastolic} is diastolic pressure in the pulmonary artery position; and wherein *PCWP_{PulsePress}* is pulse pressure in the wedge position.

11. The system of claim 10, wherein a high quality is indicated when: ${PCWP}_{Mean} < {PAP}_{Diastolic} AND a \times {PCWP}_{PulsePress} + b \times {PCWP}_{Mean} \leq c ,$ wherein *a, b, and c are* determinant values.

12. The system of claim 10 or 11, wherein a medium quality is indicated when: ${PCWP}_{Mean} < {PAP}_{Diastolic} AND a \times {PCWP}_{PulsePress} + b \times {PCWP}_{Mean} > c ,$ wherein *a, b, and c are* determinant values.

13. The system of claim 10, 11, or 12, wherein a low quality is indicated when: ${PCWP}_{Mean} \geq {PAP}_{Diastolic} .$

14. The system of any one of claims 8 to 13, wherein the one or more applications can direct the processor system to:
display the quality assessment of one or more temporal windows on the display screen.

15. The system of any one of claims 8 to 14, wherein the one or more applications can direct the processor system to:
average blood pressure measurement acquired from a wedge position of temporal windows that are determined to have a quality above a threshold to yield the PCWP measurement.

## Patentansprüche

1. Hämodynamisches Überwachungssystem (1600) zum Durchführen und Beurteilen der Qualität einer Pulmonalkapillarkeildruck-(PCWP)-Messung, das Folgendes umfasst:
einen Lungenarterienkatheter (1620), der dazu ausgelegt ist, eine Blutdruckmessung, die von einer Lungenarterienposition erfasst wird, und eine Blutdruckmessung, die von einer Keilposition erfasst wird, zu erfassen, wobei der Lungenarterienkatheter einen Ballon umfasst, der aufblasbar ist; und
Rechenverarbeitungssystem in Verbindung mit dem Lungenarterienkatheter, wobei das Rechenverarbeitungssystem Folgendes umfasst:
ein Prozessorsystem (1602);
einen Anzeigebildschirm in digitaler Verbindung mit dem Prozessorsystem; und
ein Speichersystem (1606), das eine oder mehrere Anwendungen umfasst, die das Prozessorsystem zu Folgendem veranlassen können:
Erfassen einer Blutdruckmessung in einer Position der Lungenarterie;
Aufblasen des Ballons, wobei das Aufblasen des Ballons es dem Katheter ermöglicht, in eine Keilposition zu wandern;
Erfassen einer Blutdruckmessung in der Keilposition;
Erzeugen einer Blutdruckwellenform aus einer Blutdruckmessung, die aus einer Lungenarterienposition erfasst wird, und einer Blutdruckmessung, die aus einer Keilposition erfasst wird;
Bestimmen einer PCWP-Messung aus der Blutdruckmessung aus der Keilposition;
Bestimmen einer Qualitätsbeurteilung für die PCWP-Messung unter Verwendung eines Maschinenlernmodells, das dazu ausgelegt ist, eine Qualitätsbeurteilung für die PCWP-Messung basierend auf der Blutdruckmessung, die von der Lungenarterienposition erfasst wird, und der Blutdruckmessung, die von der Keilposition erfasst wird, bereitzustellen; und
Anzeigen einer oder mehrerer der folgenden Optionen: die Blutdruckwellenform, die PCWP-Messung oder die Qualitätsbeurteilung für die PCWP-Messung auf dem Anzeigebildschirm.

2. System nach Anspruch 1, wobei die Blutdruckwellenform in Echtzeit erzeugt und auf dem Anzeigebildschirm angezeigt wird.

3. System nach Anspruch 1 oder 2, wobei die PCWP-Messung bestimmt und auf dem Anzeigebildschirm in Echtzeit angezeigt wird.

4. System nach einem der vorhergehenden Ansprüche, wobei die Qualitätsbewertung für die PCWP-Messung bestimmt und auf dem Anzeigebildschirm in Echtzeit angezeigt wird.

5. System nach einem der vorhergehenden Ansprüche, wobei die eine oder die mehreren Anwendungen das Prozessorsystem zu Folgendem veranlassen können:
vor dem Aufblasen des Ballons, Untersuchen, in Echtzeit, der Blutdruckmessung, die von einer Lungenarterienposition erfasst wurde, auf Artefakte.

6. System nach einem der vorhergehenden Ansprüche, wobei die eine oder die mehreren Anwendungen das Prozessorsystem zu Folgendem veranlassen können:
Detektieren, in Echtzeit, eines Übergangs von einer Lungenarterienposition in eine Keilposition oder von einer Keilposition in eine Lungenarterienposition durch Folgendes:
Extrahieren eines oder mehrerer hämodynamischer Merkmale aus der Blutdruckmessung in der Lungenposition und der Blutdruckmessung in der Keilposition; und
Bestimmen, unter Verwendung einer Fuzzy-Logik-Zugehörigkeitsfunktion, eines Fuzzy-Logikwerts basierend auf einer Änderung des Werts zwischen der Blutdruckmessung in der Lungenposition und der Blutdruckmessung in der Keilposition für jedes hämodynamische Merkmal des einen oder der mehreren hämodynamischen Merkmale.

7. System nach Anspruch 6, wobei das eine oder die mehreren Merkmale mittleren Druck und Pulsdruck umfassen.

8. System nach einem der vorhergehenden Ansprüche, wobei die eine oder die mehreren Anwendungen das Prozessorsystem zu Folgendem veranlassen können:
Segmentieren der Blutdruckmessung, die aus der Lungenarterienposition gewonnen wurde, und der Blutdruckmessung, die aus der Keilposition gewonnen wurde, in Zeitfenster; und
Extrahieren von Merkmalen aus den Zeitfenstern der Blutdruckmessung, die an der Lungenarterienposition erfasst wird, und der Blutdruckmessung, die an der Keilposition erfasst wird; und
Eingeben der aus den Zeitfenstern extrahierten Merkmale in das Maschinenlernmodell, um eine Qualitätsbewertung für jedes Zeitfenster zu erhalten; wobei die Qualitätsbewertung darauf basiert, ob die extrahierten Merkmale für jedes Zeitfenster als von der Lungenarterienposition oder von der Keilposition abgeleitet unterschieden werden können, um die Qualitätsbewertung für den PCWP zu erhalten, wobei das Maschinenlernmodell trainiert ist, um zu erkennen, ob ein extrahiertes Merkmal eines Zeitfensters von der an der Lungenarterienposition erfassten Blutdruckmessung oder von der an der Keilposition erfassten Blutdruckmessung abgeleitet ist.

9. System nach Anspruch 8, wobei die eine oder die mehreren Anwendungen das Prozessorsystem zu Folgendem veranlassen können:
Bestimmen, ob ein oder mehrere extrahierte Merkmale eines Zeitfensters über oder unter einem Schwellenwert liegen, um die Qualitätsbewertung für die PCWP-Messung zu erhalten.

10. System nach Anspruch 9, wobei die eine oder mehreren extrahierten Merkmale Folgendes umfassen: PCWP_{Mean,} PAP_{Diastolic} und PCWP_{PulsePress,} wobei PCWP_{Mean} ein Mittelwert von Druckmessungen ist, die in der Keilposition erfasst werden; wobei PAP_{Diastolic} diastolischer Druck in der Lungenarterienposition ist; und wobei PCWP_{PulsePress} Pulsdruck in der Keilposition ist.

11. System nach Anspruch 10, wobei eine hohe Qualität angezeigt wird, wenn:
PCWPP_{Mean} < PAP_{Diastolic} UND a × PCWP_{PulsePress} + b × PCWP_{Mean} ≤ c, wobei a, b und c Determinantenwerte sind.

12. System nach Anspruch 10 oder 11, wobei eine mittlere Qualität angezeigt wird, wenn:
PCWP_{Mean} < PAP_{Diastolic} UND a × PCWP_{PulsePress} + b × PCWP_{Mean} > c, wobei a, b und c Determinantenwerte sind.

13. System nach Anspruch 10, 11 oder 12, wobei eine niedrige Qualität angezeigt wird, wenn:${PCWP}_{Mean} \geq {PAP}_{Diastolic} .$

14. System nach einem der Ansprüche 8 bis 13, wobei die eine oder mehreren Anwendungen das Prozessorsystem zu Folgendem veranlassen können:
Anzeigen der Qualitätsbewertung eines oder mehrerer Zeitfenster auf dem Anzeigebildschirm.

15. System nach einem der Ansprüche 8 bis 14, wobei die eine oder mehreren Anwendungen das Prozessorsystem zu Folgendem veranlassen können:
durchschnittliche Blutdruckmessung, die aus einer Keilposition von Zeitfenstern erfasst wird, die bestimmt werden, eine Qualität über einem Schwellenwert aufzuweisen, um die PCWP-Messung zu erhalten.

## Revendications

1. Système de surveillance hémodynamique (1600) permettant de réaliser et d'évaluer la qualité d'une mesure de pression capillaire pulmonaire d'occlusion (PCWP), comprenant :
un cathéter d'artère pulmonaire (1620) configuré pour acquérir une mesure de pression sanguine acquise à partir d'une position d'artère pulmonaire et une mesure de pression sanguine acquise à partir d'une position d'occlusion, dans lequel le cathéter d'artère pulmonaire comprend un ballonnet qui est gonflable ; et
un système de traitement informatique en lien avec le cathéter d'artère pulmonaire, le système de traitement informatique comprenant :
un système de processeur (1602) ;
un écran d'affichage en connexion numérique avec le système de processeur ; et
un système de mémoire (1606) comprenant une ou plusieurs applications qui peuvent diriger le système de processeur pour :
acquérir une mesure de pression artérielle dans une position d'artère pulmonaire ;
gonfler le ballonnet, le gonflage du ballonnet permettant au cathéter de migrer vers une position d'occlusion ;
acquérir une mesure de pression artérielle dans la position d'occlusion ;
générer une forme d'onde de pression artérielle à partir d'une mesure de pression artérielle acquise à partir d'une position d'artère pulmonaire et d'une mesure de pression artérielle acquise à partir d'une position d'occlusion ;
déterminer une mesure PCWP à partir de la mesure de pression artérielle à partir de la position d'occlusion ; déterminer une évaluation de la qualité pour la mesure PCWP à l'aide d'un modèle d'apprentissage automatique configuré pour fournir une évaluation de la qualité pour la mesure PCWP sur la base de la mesure de pression artérielle acquise à partir de la position d'artère pulmonaire et la mesure de pression artérielle acquise à partir de la position d'occlusion ; et
afficher une ou plusieurs parmi : la forme d'onde de pression artérielle, la mesure PCWP et l'évaluation de la qualité pour la mesure PCWP sur l'écran d'affichage.

2. Système selon la revendication 1, dans lequel la forme d'onde de pression artérielle est générée et affichée sur l'écran d'affichage en temps réel.

3. Système selon la revendication 1 ou 2, dans lequel la mesure PCWP est déterminée et affichée sur l'écran d'affichage en temps réel.

4. Système selon une quelconque revendication précédente, dans lequel l'évaluation de la qualité pour la mesure PCWP est déterminée et affichée sur l'écran d'affichage en temps réel.

5. Système selon une quelconque revendication précédente, dans lequel la ou les applications peuvent diriger le système de processeur pour :
avant de gonfler le ballonnet, évaluer en temps réel la mesure de pression artérielle acquise à partir d'une position d'artère pulmonaire pour détecter des artefacts.

6. Système selon une quelconque revendication précédente, dans lequel la ou les applications peuvent diriger le système de processeur pour :
détecter en temps réel une transition d'une position d'artère pulmonaire à une position d'occlusion ou d'une position d'occlusion à une position d'artère pulmonaire par :
extraction d'une ou plusieurs caractéristiques hémodynamiques de la mesure de pression artérielle dans la position pulmonaire et de la mesure de pression artérielle dans la position d'occlusion ; et
détermination, à l'aide d'une fonction d'appartenance en logique floue, d'une valeur logique floue basée sur un changement de valeur entre la mesure de pression artérielle dans la position pulmonaire et la mesure de pression artérielle dans la position d'occlusion pour chaque caractéristique hémodynamique de la ou des caractéristiques hémodynamiques.

7. Système selon la revendication 6, dans lequel la ou les caractéristiques comprennent la pression moyenne et la pression pulsée.

8. Système selon une quelconque revendication précédente, dans lequel la ou les applications peuvent diriger le système de processeur pour :
segmenter en fenêtres temporelles la mesure de pression artérielle acquise à partir de la position d'artère pulmonaire et la mesure de pression artérielle acquise à partir de la position d'occlusion ; et
extraire des caractéristiques des fenêtres temporelles de la mesure de pression artérielle acquise à partir de la position d'artère pulmonaire et de la mesure de pression artérielle acquise à partir de la position d'occlusion ; et
entrer les caractéristiques extraites des fenêtres temporelles dans le modèle d'apprentissage automatique pour fournir une évaluation de la qualité pour chaque fenêtre temporelle ; dans lequel l'évaluation de la qualité est basée sur le fait que les caractéristiques extraites pour chaque fenêtre temporelle peuvent être différenciées comme dérivées de la position d'artère pulmonaire ou de la position d'occlusion pour fournir l'évaluation de la qualité pour le PCWP, le modèle d'apprentissage automatique étant entraîné pour détecter si une caractéristique extraite d'une fenêtre temporelle est dérivée de la mesure de pression sanguine acquise à partir de la position d'artère pulmonaire ou de la mesure de pression artérielle acquise à partir de la position d'occlusion.

9. Système selon la revendication 8, dans lequel la ou les applications peuvent diriger le système de processeur pour :
déterminer si une ou plusieurs caractéristiques extraites d'une fenêtre temporelle sont au-dessus ou au-dessous d'un seuil pour obtenir l'évaluation de la qualité pour la mesure PCWP.

10. Système selon la revendication 9, dans lequel les une ou plusieurs caractéristiques extraites comprennent : PCWP_{Mean}, PAP_{Diastolic}, et PCWP_{PulsePress}, où PCWP_{Mean} est une valeur moyenne des mesures de pression acquises dans la position d'occlusion ; où PAP_{Diastolic} est la pression diastolique dans la position d'artère pulmonaire ; et où PCWP_{PulsePress} est la pression pulsée dans la position d'occlusion.

11. Système selon la revendication 10, dans lequel une qualité élevée est indiquée lorsque :
PCWPP_{Mean} < PAP_{Diastolic} ET a × PCWP_{PulsePress} + b × PCWP_{Mean} ≤ c, où a, b et c sont des valeurs déterminantes.

12. Système selon la revendication 10 ou 11, dans lequel une qualité moyenne est indiquée lorsque :
PCWP_{Mean} < PAP_{Diastolic} ET a × PCWP_{PulsePress} + b × PCWP_{Mean} > c, où a, b et c sont des valeurs déterminantes.

13. Système selon la revendication 10, 11 ou 12, dans lequel une qualité faible est indiquée lorsque :${PCWP}_{Mean} \geq {PAP}_{Diastolic} .$

14. Système selon l'une quelconque des revendications 8 à 13, dans lequel la ou les applications peuvent diriger le système de processeur pour :
afficher l'évaluation de la qualité d'une ou de plusieurs fenêtres temporelles sur l'écran d'affichage.

15. Système selon l'une quelconque des revendications 8 à 14, dans lequel la ou les applications peuvent diriger le système de processeur pour :
calculer la moyenne de la mesure de la pression artérielle acquise à partir d'une position d'occlusion de fenêtres temporelles dont la qualité est déterminée comme étant supérieure à un seuil pour produire la mesure PCWP.
